Europäisches Patentamt

European Patent Office

Office européen des brevets

(19)

(11) EP 0 990 002 B1

# (12) FASCICULE DE BREVET EUROPEEN

(45) Date de publication et mention
de la délivrance du brevet:
**05.10.2005 Bulletin 2005/40**

(21) Numéro de dépôt: **98959962.6**

(22) Date de dépôt: **11.12.1998**

(51) Int Cl.$^7$: **C08B 37/02**, A61K 31/715,
A61K 31/725

(86) Numéro de dépôt international:
**PCT/FR1998/002699**

(87) Numéro de publication internationale:
**WO 1999/029734 (17.06.1999 Gazette 1999/24)**

(54) **DERIVES DE DEXTRANE, LEUR PROCEDE DE PREPARATION ET LEURS APPLICATIONS COMME MEDICAMENTS A ACTION BIOLOGIQUE SPECIFIQUE**

DEXTRANDERIVATE, VERFAHREN ZU DEREN HERSTELLUNG UND VERWENDUNGEN ALS ARZNEIMITTEL MIT SPEZIFISCHER, BIOLOGISCHER WIRKUNG

DEXTRAN DERIVATIVES, PREPARATION METHOD AND APPLICATIONS AS MEDICINE WITH SPECIFIC BIOLOGICAL ACTION

(84) Etats contractants désignés:
**AT BE CH CY DE DK ES FI FR GB GR IE IT LI LU MC NL PT SE**

(30) Priorité: **11.12.1997 FR 9715702**

(43) Date de publication de la demande:
**05.04.2000 Bulletin 2000/14**

(73) Titulaire: **BIODEX**
**59230 St Amand les Eaux (FR)**

(72) Inventeurs:
- **CHAUBET, Frédéric**
  **95600 Eaubonne (FR)**
- **HUYNH, Rémi**
  **77500 Chelles (FR)**
- **DAHRI, Latifa**
  **F-92250 La Garenne Colombes (FR)**
- **CORREIA, José**
  **F-28100 Dreux (FR)**
- **JOZEFOWICZ, Marcel**
  **F-60260 Lamorlaye (FR)**
- **JOZEFONVICZ, Jacqueline**
  **F-60260 Lamorlaye (FR)**

(74) Mandataire: **Goulard, Sophie et al**
**Cabinet ORES,**
**36,rue de St Pétersbourg**
**75008 Paris Cedex (FR)**

(56) Documents cités:
**EP-A- 0 023 854**     **EP-A- 0 146 455**
**WO-A-90/10456**     **WO-A-91/12011**
**DE-B- 1 152 396**     **FR-A- 2 651 436**

- **O. MAIGA-REVEL ET AL.: "New investigations on heparin-like derivatized dextrans: CMDBS, synergistic role of benzylamide and sulfate substituents in anticoagulant activity" CARBOHYDRATE POLYMERS, vol. 32, 1997, pages 89-93, XP000686949 cité dans la demande**
- **CHEMICAL ABSTRACTS, vol. 78, no. 1, 8 janvier 1973 Columbus, Ohio, US; abstract no. 4485, XP002076097 & JP 47 020117 A (SEIKAGAKU KOGYO CO. LTD.) 27 septembre 1972**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Jouve, 75001 PARIS (FR)

**Description**

La présente invention est relative à des dérivés de dextrane, et à leurs applications comme médicaments à action biologique spécifique, telle qu'une action cicatrisante, une action anti-complémentaire (substitut du plasma), une action modulatrice de la prolifération ou une action anticoagulante et plus spécifiquement une action anti-thrombotique, ainsi qu'à leur procédé de préparation.

Différents dextranes substitués par des chaînes latérales portant des groupes carboxylates et sulfonates ont été décrits. En particulier le Brevet français 2 461 724 et le Brevet français 2 555 589 décrivent des dextranes substitués par lesdits groupes, présentant respectivement des propriétés anticoagulantes et des propriétés anticoagulantes et antiinflammatoires; le Brevet européen 0 462 194 décrit les propriétés de régénération cellulaire et tissulaire de tels dextranes substitués.

Il a également été montré que de tels dextranes substitués pouvaient présenter d'autres activités biologiques, en fonction du taux de substitution par lesdits groupes ; en particulier, le Brevet européen 0 514 449 décrit des dextranes (D) substitués par des groupes carboxyméthyle (CM) et carboxyméthylbenzylamide sulfonate (BS) de formule générale $D_X CM_Y BS_Z$, dans laquelle X, qui représente le nombre moyens d'unités saccharidiques non substituées pour 100 unités saccharidiques, est inférieur ou égal à 50, Y, qui représente le nombre moyen de groupes carboxyméthyle pour 100 unités saccharidiques, est compris entre 10 et 90 et Z, qui représente le nombre moyen de groupes carboxyméthylbenzylamide sulfonate pour 100 unités saccharidiques, est compris entre 15 et 35, pour l'obtention d'un agent inhibiteur de la croissance des cellules tumorales.

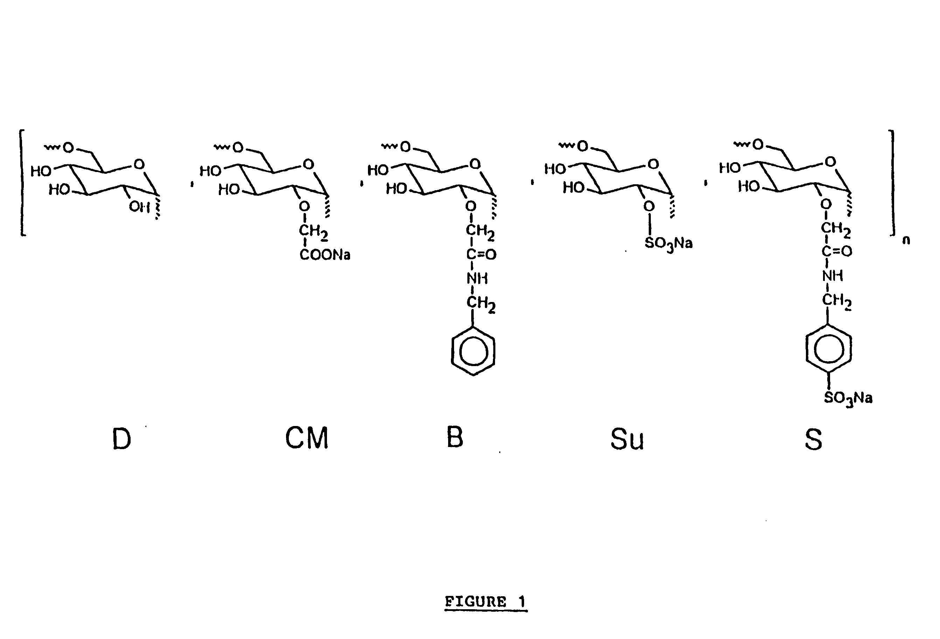

Ces différents dérivés, dont la structure est résumée sur la figure 1, sont généralement obtenus par substitution statistique du dextrane avec trois groupes différents : carboxyméthyle (CM), carboxyméthylbenzylamide (B) et carboxyméthylbenzylamide sulfonate (S) (sulfonatation sur le noyau aromatique par l'acide chlorosulfonique).

De manière plus précise :

a) la carboxyméthylation du dextrane (production de CMD) est réalisée en milieu aqueux basique, par action de l'acide monochloroacétique. Trois réactions successives de carboxyméthylation sont nécessaires pour obtenir un degré de substitution du dextrane (ds), exprimé par rapport au nombre de fonctions hydroxyles libres dans une unité glucosidique du dextrane, compris entre 0,7 et 1,1 ;

b) le couplage de la benzylamine sur les groupes carboxyméthyles (production de CMDB) est fondé sur l'aptitude de la fonction carboxylate à former un anhydride mixte instable capable de réagir avec un réactif portant une fonction amine primaire (R-NH$_2$). Deux procédés différents ou réactions d'activation ont été utilisés pour aboutir à la formation d'un anhydride mixte :

- action du chloroformate d'isobutyle (CIB) ou
- action du N-éthoxycarbonyl-2-éthoxy-1,2-dihydroquinoléine (EEDQ).

Dans les deux cas, la réaction est mise en oeuvre dans un milieu hétérogène, respectivement eau/diméthylformamide ou eau/éthanol.

Ces deux procédés de couplage donnent des degrés de substitution (ds) semblables, avoisinant 0,08 à 0,12, en une seule étape. Dans le cas du couplage par l'EEDQ, le ds peut atteindre 0,25 à 0,30, en une seule étape, lorsque l'on procède à l'activation du produit intermédiaire à une température de 30 à 40°C.

Comme pour la carboxyméthylation, pour atteindre des ds de benzylamine élevés, il n'est pas possible d'augmenter les concentrations des différents réactifs. Il est donc nécessaire de faire des couplages successifs pour améliorer le rendement de la réaction. Le CMDB précipité, lavé et séché après le premier couplage va subir un second et/ou un troisième couplage exactement dans les mêmes conditions que le premier, sans considération des substitutions dues au premier couplage.

c) La sulfonatation ou la sulfatation se fait par action de l'acide monochlorosulfonique sur le CMDB, en milieu organique anhydre (dichlorométhane, par exemple) et en phase hétérogène, le CMDB n'étant pas soluble dans le dichlorométhane. Dans un tel milieu hétérogène, la distribution des sulfates au niveau des unités saccharidiques se fait de façon inégale (RICKETTS, C.R., *J. Chem. Soc.,* 1956, 3752-3756). Il est nécessaire d'opérer la réaction en excès d'acide monochlorosulfonique, tout en évitant une hydrolyse acide de la chaîne polysaccharidique.

Les rapports [HSO$_3$Cl]/[unités B fixées] pour les CMDB et [HSO$_3$Cl]/[OH libres] pour le CMD varient entre 0,8 et 3.

Pour obtenir essentiellement une sulfonatation des noyaux aromatiques (unités B), le rapport molaire [HSO$_3$Cl]/[unités B fixées] doit être égal à 3, alors que pour obtenir une sulfatation des fonctions hydroxyles portées par les unités glucosidiques (production de fonctions sulfates), le rapport molaire [HSO$_3$Cl]/[OH libres] est de l'ordre de 1. Dans tous les cas, la concentration en acide chlorosulfonique dans le milieu réactionnel ne doit pas dépasser 0,15 M. Dans ces conditions, le pourcentage d'unités portant une fonction sulfonate dépend du pourcentage d'unités substituées par des groupes benzylamide. Une étude récente (MAIGA-REVEL O. *et al., Carbohydrates Polymers,* 1997, 32, 89-93) a montré que l'activité anticoagulante des dérivés de dextrane CMDSu (=carboxyméthyldextrane sulfate) et CMDBS (=carboxyméthyldextrane benzylamide sulfonate) de l'Art antérieur dépend de leur teneur en soufre; toutefois, les CMDBS présentent une activité anticoagulante supérieure à celle obtenue avec les CMDSu, pour une teneur en soufre identique.

Les dérivés de dextrane obtenus dans les conditions définies ci-dessus, ont l'inconvénient de présenter une distribution irrégulière des groupements chimiques et des tailles des chaînes polysaccharidiques, conduisant à un produit final hétérogène, dont il est difficile de contrôler les propriétés.

Les Inventeurs ont mis au point un nouveau procédé de préparation de dérivés de dextrane, qui permet de mieux maîtriser la production de produits spécifiquement définis (degré de substitution contrôlable, homogénéité de la distribution des groupements chimiques chargés ou non et homogénéité en taille des chaînes polysaccharidiques du produit final, sélection et meilleure reproductibilité de l'activité recherchée).

La présente invention a pour objet des dérivés de dextrane de formule générale $DCM_aB_bSu_cS_d$, dans laquelle :

D représente une chaîne polysaccharidique, de préférence constituée par des enchaînements d'unités glucosidiques,

CM représente des groupes carboxyrnéthyle,

B représente des groupes carboxyméthylbenzylamides,

Su représente des groupes sulfates (sulfatation des fonctions hydroxyles libres portées par les unités glucosidiques),

S représente des groupes sulfonates (sulfonatation des noyaux aromatiques),

$\underline{a}$, $\underline{b}$, $\underline{c}$ et $\underline{d}$ représentent le degré de substitution (ds), exprimé par rapport au nombre de fonctions hydroxyles libres dans une unité glucosidique du dextrane, respectivement en groupements CM, B, Su et S ; $\underline{a}$ étant ≥ à 0,3, $\underline{b}$ étant égal à 0 ou ≥ à 0,1, $\underline{c}$ étant égal à 0 ou ≥ à 0,1 et $\underline{d}$ étant égal à 0 ou ≤ à 0,15, à condition que lorsque $\underline{d}$ = 0, $\underline{a}$ et $\underline{b}$ sont ≠ 0,

lesquels produits présentent :

- une homogénéité de la distribution des tailles de chaînes, illustrée par un profil d'élution de type gaussien symétrique en chromatographie d'exclusion stérique haute performance, et
- une homogénéité de la distribution des groupements chimiques chargés, illustrée par un profil d'élution à un seul pic symétrique en chromatographie d'échange d'ions basse pression.

On considère ces produits comme étant des copolymères constitués de sous-unités fictives R-OH et R-OX, X pouvant être un groupement carboxyméthyle (CM), benzylamide (B), sulfate (Su) ou sulfonate (S), la chaîne polysaccharidique du dextrane non substitué étant considérée comme étant constituée de 300 sous-unités fictives R-OH, au lieu de 100 unités glucosidiques, eu égard au fait qu'une unité glucosidique non substituée comporte trois groupes hydroxyles libres. Ainsi, un dextrane carboxyméthyle (DCM) avec un degré de substitution (ds) de 1,2 en groupements carboxyméthyle contient 1,20 groupement substitué (R-CM) et 1,80 groupement hydroxyle libre (R-OH), par unité glucosidique.

On obtient ainsi, et ce, contrairement aux produits hétérogènes de l'Art antérieur, des produits homogènes, de composition ciblée, dans lesquels les groupements chimiques bioactifs sont distribués le long des chaînes macromoléculaires selon un ordonnancement spécifique, conférant au produit une propriété biologique que l'on ne retrouvera pas sur un produit de même composition globale, mais dans lequel la répartition desdits groupements le long des chaînes macromoléculaires est différente (préparation différente, notamment).

En d'autres termes, dans les dérivés de dextrane selon l'invention, la distribution des groupements chimiques confère au produit final une propriété biologique spécifique ; une telle distribution a pour conséquence que la composition chimique de chaque chaîne polysaccharidique est identique à la composition chimique globale du produit. De ce fait, il existe une composition chimique optimale pour une activité biologique spécifique maximale ; il y a donc une relation directe entre la propriété biologique considérée et la composition chimique globale du produit.

Par exemple :

- les dérivés de dextrane, de formule générale $DCM_aB_bSu_cS_d$, telle que définie ci-dessus, dans laquelle $\underline{a}$ ≥ 0,6, $\underline{b}$ ≠ 0, $\underline{c}$ égal à 0 ou ≤ 0,5 et $\underline{d}$ ≤ 0,15 ou d égal à 0 sont essentiellement des agents cicatrisants, de préférence lorsqu'ils ont une masse molaire comprise entre 3.000 et 500.000 g/mole ; les dérivés de dextrane préférés en tant qu'agents cicatrisants sont ceux dans lesquels $\underline{a}$ est compris entre 0,7 et 0,9 ; $\underline{c}$ ≤ 0,5 et $\underline{d}$ ≤ 0,15 ou égal à 0 ;
- les dérivés de dextrane, de formule générale $DCM_aB_bSu_cS_d$, telle que définie ci-dessus, dans laquelle $\underline{a}$ ≥ 0,3, $\underline{b}$

≠ 0, $\underline{c}$ égal à 0 ou ≤ 0,4 et $\underline{d}$ ≤ 0,15 ou égal à 0 sont essentiellement des agents à activité anti-complémentaire et substitut du plasma, de préférence lorsqu'ils ont une masse molaire comprise entre 10.000 et 60.000 g/mole ; les dérivés de dextrane préférés en tant qu'agents à activité anti-complémentaire et substitut du plasma sont ceux dans lesquels $\underline{a}$ est compris entre 0,40 et 1,15, $\underline{b}$ ≤ 0,4, $\underline{c}$ ≤ 0,2 et $\underline{d}$ ≤ 0,15 ou égal à 0 ;

- les dérivés de dextrane, de formule générale $DCM_aB_bSu_cS_d$, telle que définie ci-dessus, dans laquelle $\underline{a}$ ≥ 0,5, $\underline{b}$ ≠ 0, $\underline{c}$ égal à 0 ou ≤ 0,4 et $\underline{d}$ ≤ 0,15 sont essentiellement des agents modulateurs de la prolifération cellulaire, de préférence lorsqu'ils ont une masse molaire comprise entre 3.000 et 100.000 g/mole ; les dérivés de dextrane préférés en tant qu'agents modulateurs de la prolifération cellulaire sont ceux dans lesquels $\underline{a}$ est compris entre 0,5 et 1,2 ; $\underline{b}$ est compris entre 0,2 et 0,6 ; $\underline{c}$ est compris entre 0,1 et 0,4 et $\underline{d}$ ≤ 0,15 ou égal à 0 ; et

- les dérivés de dextrane, de formule générale $DCM_aB_bSu_cS_d$, telle que définie ci-dessus, dans laquelle $\underline{a}$ ≥ 0,4, $\underline{c}$ ≥ 0,3 et $\underline{d}$ ≤ 0,15 ou égal à 0 sont essentiellement des agents anti-coagulants, de préférence lorsqu'ils ont une masse molaire comprise entre 3.000 et 20.000 g/mole et une valeur de $\underline{b}$ ≠ 0.

La présente invention a également pour objet des médicaments, caractérisés en ce qu'ils comprennent comme principe actif au moins un dérivé de dextrane tel que défini ci-dessus, éventuellement associé à un autre principe actif et/ou à au moins un véhicule pharmaceutiquement acceptable et/ou un support physiologiquement acceptable, de préférence un liposome.

Les principes actifs associés sont choisis dans le groupe qui comprend les dextranes, les facteurs de croissance (par exemple un facteur de croissance de fibroblaste (FGF) acide ou un FGF basique), les anesthésiques locaux, les antiinfectieux, les protéines sériques et le collagène.

La présente invention a également pour objet un procédé de préparation (procédé 1) de dérivés du dextrane de formule générale $DCM_aB_bSu_c,S_d$, telle que définie ci-dessus, caractérisé en ce qu'il comprend les étapes suivantes :

a) **carboxyméthylation** comprenant (i) l'activation d'un dextrane non substitué, par mise en contact dudit dextrane avec un milieu hydro-alcoolique liquide biphasique basique pendant au moins 1 h sous agitation, (ii) addition de l'acide monochloroacétique au produit activé obtenu, à une température comprise entre 40 et 90°C, de préférence à 60°C, le rapport $R_{CM}$, égal au nombre de moles d'acide monochloracétique/nombre de moles d'OH étant compris entre 0,3 et 2, (iii) isolement et éventuellement purification du dextrane carboxyméthyle (DCM) obtenu.

b) **couplage de la benzylamine sur les groupes carboxyméthyle** (benzylamidification) comprenant (i) la mise en contact, pendant au moins 2 h et en milieu aqueux acide, du DCM obtenu en a) avec une amine primaire (benzylamine), en présence d'une carbodiimide hydrosoluble telle que le 1-cyclohexyl-3-(2-morpholinoéthyl)-carbodiimide méta-*p*-toluène sulfonate (CMC) ou le chlorohydrate de 1-éthyl-3-(3-diméthylamino-propyl)-carbodiimide (EDC) comme agent de couplage, à une température comprise entre 0°C et 30°C, le rapport molaire CMC/CM étant compris entre 0,25 et 2 et le rapport molaire benzylamine/CM étant compris entre 0,25 et 2, (ii) l'isolement du dextrane carboxyméthyl benzylamide (DCMB) obtenu et éventuellement sa purification.

Une telle étape, réalisée en milieu homogène et en présence d'une carbodiimide hydrosoluble comme réactif de couplage permet de mieux maîtriser la réaction et donc la préparation du produit final, ce dernier présentant une homogénéité de la distribution des tailles de chaînes, illustrée par un profil d'élution de type gaussien symétrique en chromatographie d'exclusion stérique haute performance et une homogénéité de la distribution des groupements chimiques chargés, illustrée par un profil d'élution à un seul pic symétrique en chromatographie d'échange d'ions basse pression.

c) **sulfatation** comprenant (i) la formation d'un sel de trialkylammonium du DCMB obtenu en b), (ii) la solubilisation du sel obtenu dans un solvant polaire anhydre, généralement une base de Lewis (donneur d'électron), telle que le diméthylsulfoxyde (DMSO) ou la diméthylformamide (DMF) et (iii) l'ajout audit sel en solution, d'un complexe à base de trioxyde de soufre tel que $SO_3$-pyridine, $SO_3$-triéthylamine ou $SO_3$-DMF en solution dans le même solvant, à une température inférieure à 70°C, le rapport molaire complexe à base de trioxyde de soufre/OH libres étant compris entre 0,25 et 12, et éventuellement

d) **sulfonatation** des groupes B par mélange sous agitation d'un dérivé DCMBSu en suspension dans un solvant anhydre avec de l'acide chlorosulfonique en solution dans le même solvant, à une température comprise entre la température ambiante et la température d'ébullition du solvant utilisé.

De manière inattendue, le procédé selon l'invention permet de contrôler le degré de substitution du dextrane, en un nombre d'étapes significativement inférieur au nombre d'étapes des procédés de l'Art antérieur, pour obtenir des produits ayant un profil d'élution de type gaussien symétrique en chromatographie d'exclusion stérique haute performance et un profil d'élution à un seul pic symétrique en chromatographie d'échange d'ions basse pression et l'activité biologique recherchée, avec des rendements significativement supérieurs à ceux de l'Art antérieur.

En outre, le procédé selon l'invention permet de synthétiser de façon reproductible un produit de composition chimique souhaitée pour la propriété biologique retenue. Ainsi à une composition chimique donnée d'un produit correspond préférentiellement une activité biologique.

Par exemple, on obtient à l'étape a), en une seule étape, un ds en CM de 1,0±0,1 par unité glucosidique et un rendement supérieur ou égal à 80 %, pour une valeur de $R_{CM}$= 0,85, dans un milieu mixte eau-tertiobutanol ou eau-isopropanol (15/85, v/v), sous agitation pendant 2 heures à 60°C ; on obtient, à l'étape b), à partir d'un DCM de ds = 1 en CM, pour un rapport molaire CMC/CM de 0,75 et un rapport molaire benzylamine/CM de 1, et sous agitation à température ambiante pendant 16 heures, un produit DCMB ayant un ds en CM de 0,70±0,05 et un ds en B de 0,30±0,03, avec un rendement supérieur à 80 % ; et on obtient , à l'étape c), un rendement supérieur ou égal à 60 %.

Selon un mode de mise en oeuvre avantageux dudit procédé, à l'étape a), le rapport eau:alcool est compris entre 10:90 (v/v) et 25:75 (v/v), et est de préférence de 15:85 (v/v).

En variante, le procédé de préparation (procédé 2) des dérivés de dextrane de formule générale $DCM_aB_b$-$Su_cS_d$, telle que définie ci-dessus, dans laquelle $\underline{a} \neq 0$, $\underline{b}$ est différent de 0 et $\underline{d}$ = 0, comprend les étapes suivantes :

a) préparation de N-méthyl-phényl-2-chloroacétamide par mise en contact d'une benzylamine avec du chlorure de chloroacétyle, puis isolement et purification du produit,

b) mise en contact du dextrane en solution hydro-alcoolique basique avec successivement la N-méthyl-phényl-2-chloroacétamide en solution alcoolique obtenu en a) en présence d'acide monochloroacétique en solution alcoolique, maintien du mélange obtenu à une température supérieure à 40°C, de préférence à 60°C, puis isolement et éventuellement purification du DCMB obtenu, et

c) sulfatation du produit obtenu en b) comprenant (i) la formation d'un sel de trialkylammonium, (ii) la solubilisation du sel obtenu dans un solvant polaire anhydre, généralement une base de LEWIS, telle que le diméthylsulfoxyde (DMSO) ou la diméthylformamide (DMF), (iii) l'ajout audit sel en solution d'un complexe à base de trioxyde de soufre tel que $SO_3$-pyridine, $SO_3$-triéthylamine ou $SO_3$-DMF en solution dans le même solvant, à température inférieure à 70°C, le rapport molaire complexe à base de trioxyde de soufre/OH libres étant compris entre 0,25 et 12 et (iv) l'isolement et éventuellement la purification du DCMBSu obtenu.

La présence des étapes b) et c) ci-dessus permet d'obtenir la même homogénéité du produit final que celle obtenue avec le procédé 1 ci-dessus.

Pour préparer des dérivés de dextrane de formule générale $DMC_aB_bSu_cS_d$, telle que définie ci-dessus, dans laquelle $\underline{a}$ est différent de 0, $\underline{b}$ = 0 et $\underline{d}$ = 0 (→DMCSu) (procédé 3), ledit procédé est caractérisé en ce qu'il comprend les étapes suivantes :

a) **carboxyméthylation** d'un dextrane non substitué, dans les conditions exposées ci-dessus et
b) **sulfatation** du DMC obtenu en a) dans les conditions exposées ci-dessus.

Outre les dispositions qui précèdent, l'invention comprend encore d'autres dispositions, qui ressortiront de la description qui va suivre, qui se réfère à des exemples de mise en oeuvre du procédé objet de la présente invention ainsi qu'aux dessins annexés, dans lesquels :

- la figure 1 illustre schématiquement la structure d'un dextrane substitué par les différents groupements chimiques fixés sur les unités glucosidiques ; la position du substituant sur les différentes carbones des unités de base glucosidiques est présentée en 2, à titre d'exemple ;

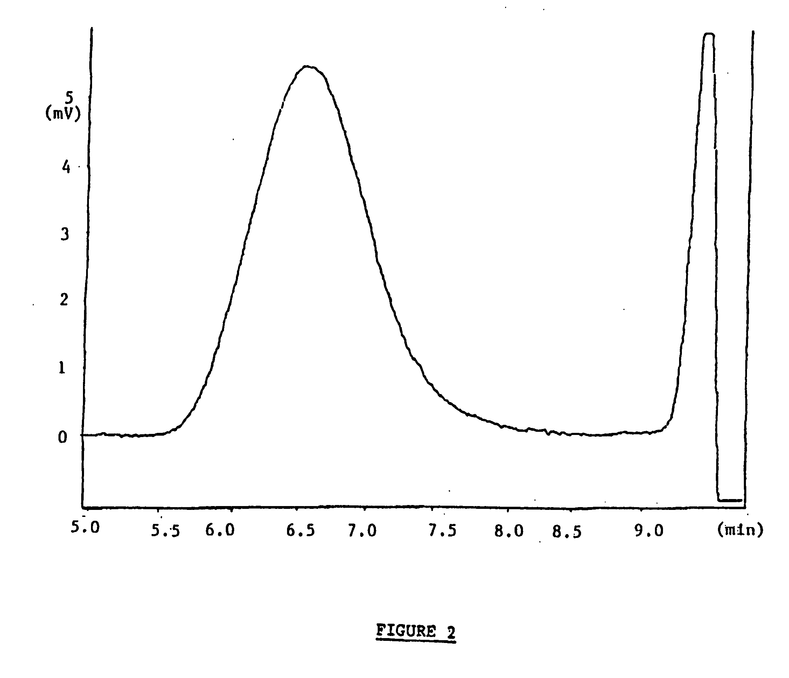

- la figure 2 illustre le chromatogramme d'exclusion stérique haute performance obtenu avec un dérivé de dextrane tel que défini ci-dessus (DMCBSu);

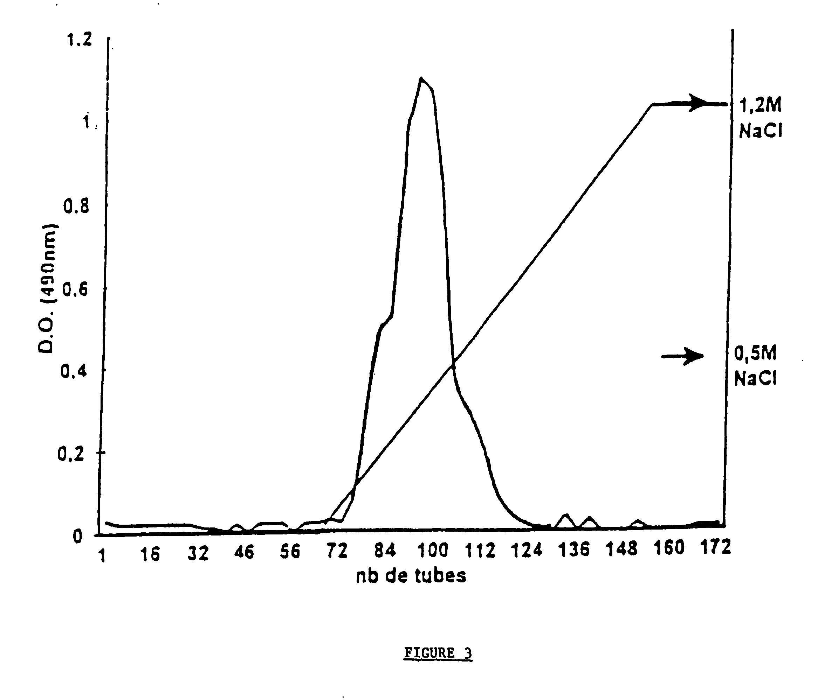

- la figure 3 illustre le chromatogramme d'échange d'ions obtenu avec un dérivé de dextrane tel que défini ci-dessus (DMCBSu).

Il doit être bien entendu, toutefois, que ces exemples sont donnés uniquement à titre d'illustration de l'objet de l'invention, dont ils ne constituent en aucune manière une limitation.

**<u>EXEMPLE 1</u> : Caractérisations physico-chimiques des produits obtenus.**

**\* <u>Dosage acide-base</u>**

Le dosage acidimétrique donne une estimation du nombre de milliéquivalents de groupements carboxyméthyles et permet d'établir la composition des produits finaux.

**\* Protocole**

Un étalonnage titrant permet de déterminer la molarité de la soude à l'aide d'un standard : le monohydrogénophtalate de potassium.

Entre 15 et 25 mg de produit purifié, séché (sous vide à 40°C) et pesé très précisément sont dissous dans un mélange eau-acétone (v:v, 50:50), de façon à avoir une solution de concentration finale d'environ 0,25 mg/ml.

Le pH apparent, alors compris entre 7 et 8, est abaissé entre 2,8 et 3, à l'aide d'une solution de $HNO_3$ 10 %. L'addition de la solution titrante est effectuée par une microburette automatique de précision d'un volume total de 5 ml. Sur chaque produit, au moins 3 dosages sont réalisés et le résultat, exprimé en milliéquivalents de fonctions carboxyméthyles par gramme de polymère, permet de calculer le taux de substitution en groupements carboxyméthyles. Cette méthode permet aussi, par différence du nombre de milliéquivalents de fonctions carboxyméthyles avant et après benzylamidification, d'estimer le taux de substitution en motif B. Pour l'ensemble des produits, les compositions ont été établies à l'aide de l'analyse élémentaire.

**\* Analyse élémentaire de l'azote, du soufre et du chlore**

Le dosage de l'azote et du soufre par analyse élémentaire, exprimé en gramme d'élément pour 100 g de produit, permet de déterminer respectivement le pourcentage de substitution des groupements Benzylamide (B), Sulfonate (S) et Sulfate (Su).

Le dosage du chlore permet de vérifier le degré de pureté des échantillons, le pourcentage de sel (NaCl) ne devant pas excéder 1 à 2 % au terme de la purification.

**\* Désulfatation des produits (DCMBSuS)**

Le produit sous forme de sel de sodium (250 mg, 10 ml) est agité lentement à température ambiante avec 3 ml de résine échangeuse de cations (Amberlite IR120 H+, 16-45 mesh, capacité d'échange totale : 1,9 meq/ml). Après 2 h, la solution acide est filtrée, neutralisée avec de la pyridine (1 à 2 ml) jusqu'à un pH de 6-6,5 et évaporée à sec. Le sel de pyridinium obtenu est repris 3 fois avec 10 ml de méthanol anhydre et évaporé à sec.

Le résidu est dispersé dans 25 ml d'un mélange « 90:9:1 » en diméthylsulfoxyde (DMSO), méthanol et pyridine. La solution est mise à agiter dans un bain d'huile chauffé à 90°C pendant 72 h. La réaction est arrêtée en ajoutant 20 ml d'eau bidistillée froide, puis le mélange est neutralisé avec une solution aqueuse de NaOH 1M. Le produit désulfaté est purifié par chromatographie d'exclusion stérique basse pression sur une colonne Sephadex G15 puis diafiltré sur cellule équipée d'une membrane de seuil de coupure 1000 Da. Entre 160 et 210 mg de produit désulfaté sont obtenus.

**\* Détermination de la masse molaire**

La masse molaire des produits préparés est déterminée par chromatographie d'exclusion stérique haute performance (HPSEC). Trois systèmes de colonnes ont été utilisés :

- deux colonnes montées en série (Si300 diol et Hema Sec Bio 40) couvrant un domaine de fractionnement compris entre $10^6$ et 5 000 g/mol,
- une colonne Zorbax GF450 (domaine d'exclusion : 450 000 - 20 000 g/mol),
- une colonne TSK gel G2000 (domaine d'exclusion : 40 000 - 1 000 g/mol).

Elles sont étalonnées avec un kit de pullulanes (853 000 à 5 800 g/mol) ; le dextrane enzymatique (oligosaccharide, 1 500 g/mol) ; le mélezitose (trisaccharide, 522 g/mol) ; le sucrose (disaccharide, 342 g/mol) et le glucose (monosaccharide, 180 g/mol). Les échantillons à analyser sont préparés (à 2 mg/mL) et élués avec une solution de NaCl 0,15 M, $Na_2HPO_4$ 0,05 M tamponnée à pH 7,3. Après filtration sur filtre de porosité 0,2 µm, 100 µl de l'échantillon sont injectés, le débit étant réglé par une pompe Waters modèle 510 et la détection à la sortie de colonne se faisant par réfractométrie différentielle. Les chromatogrammes traités par le logiciel d'analyse GPC Chromstar permettent de déterminer la masse molaire chromatographique (Mc), les masses molaires moyennes en poids ($\overline{Mp}$) et en nombre ($\overline{Mn}$) ainsi que l'indice de polymolécularité des produits.

**\* Spectroscopie infrarouge à transformé de Fourier (FTIR)**

1 mg de produit à analyser est mélangé à une solution de 150 mg de KBr dans 1 ml d'eau bidistillée. L'ensemble est filtré sur un filtre de porosité 0,45 µm, congelé et lyophilisé ; une pastille est préparée avec la poudre obtenue et le spectre est aussitôt enregistré. L'appareil est un spectrophotomètre FTIR (Perkin Elmer modèle 1600) et les spectres sont traités sur ordinateur avec le logiciel IRDM (Perkin Elmer).

**EXEMPLE 2 : Préparation de différents DCMBSu avec b = 0 ou b ≠ 0.**

Le Tableau I suivant présente les différents dérivés obtenus et leurs propriétés établies après analyse du produit final.

TABLEAU I

| Produits | Conditions de synthèse | Composition Degré de substitution ± 10 % | | | Masse molaire | Propriét biologique majeure |
|---|---|---|---|---|---|---|
| | | CM | B | Su | (g/mole) | |
| DCMBSu1 | a | 0,75 | 0,20 | 0,15 | 48000 | SP |
| DCMBSu2 | b | 0.80 | 0,25 | 0,15 | 70 000 | SP |
| DCMBSu3 | c | 1,10 | 0,40 | 0,40 | 59 000 | C |
| DCMBSu4 | d | 0,70 | 0,30 | 0,25 | 48 000 | SP |
| DCMBSu5 | e | 0,80 | 0,35 | 0,15 | 56 000 | MP |
| DCMBSu6 | f | 0,60 | 0,50 | 0,30 | 63 000 | MP |
| DCMBSu7 | g | 0,70 | 0,30 | 0,16 | 70 000 | MP |
| DCMBSu8 | h | 0,70 | 0,20 | 0,35 | 67 000 | SP |
| DCMSu | i | 1,00 | 0 | 0,37 | 70 000 | AC |
| SP = substitut du plasma, C = cicatrisant, AC = anti-coagulant, MP = modulateur de la prolifération cellulaire. | | | | | | |

a) DCMBSu1

Carboxyméthylation

Dans un réacteur double enveloppe de 2 l maintenu à température ambiante et muni d'un système d'agitation, on disperse 50 g (0,31 mole) de dextrane T40 de masse molaire de 40 000 environ dans 612 ml d'isopropanol.

Dans un bécher de 500 ml, on dissout 67,9 g (1,7 moles) de NaOH dans 188 ml d'eau bidistillée. La solution obtenue est refroidie à 4°C et ajoutée lentement au mélange dextrane-isopropanol. L'ensemble est laissé sous agitation pendant 1 heure.

Dans un bécher de 1 l, on dissout 72,9 g (0,77 mole) d'acide monochloroacétique dans 450 ml d'isopropanol (rapport molaire acide/OH=2,5).

La solution d'acide monochloroacétique est ajoutée rapidement dans le réacteur double enveloppe et l'ensemble est porté à 60°C à l'aide d'un thermostat relié au réacteur. L'ensemble est maintenu à cette température, sous agitation, pendant 1h30.

La phase aqueuse est ensuite récupérée et diluée jusqu'à obtenir un volume de 2,5 l. La solution ainsi obtenue est purifiée à volume constant sur une membrane de seuil de coupure 5 000 g/mole, avec de l'eau bidistillée jusqu'à obtenir une conductivité du filtrat satisfaisante. La concentration de la solution est de 28 g/l. 20 ml de cette solution sont congelés et lyophilisés pour les analyses.

On obtient 70 g (0,289 mole) de DCM avec un degré de substitution en CM de 1,0 ± 0,1.

Benzylamidification

Le pH de la solution précédente est ajusté à 4,75. Le mélange est refroidi à 4°C. On y ajoute rapidement 62,3 g d'agent de couplage (CMC) (rapport molaire CMC/CM=0,5). Dès que l'agent de couplage est entièrement solubilisé dans le mélange, on ajoute rapidement 16 ml de benzylamine (rapport molaire benzylamine/CM=0,5). Le mélange est ensuite laissé sous agitation pendant 16 heures à la température ambiante.

Le mélange est ensuite ultrafiltré à volume constant sur une membrane de seuil de coupure 5 000 g/mole, successivement avec 15 1 d'eau osmosée jusqu'à pH 7, puis avec 10 l de tampon carbonate 0,05 M pH 9,6, et enfin avec de l'eau bidistillée jusqu'à une conductivité du filtrat satisfaisante. La concentration de cette solution est de 21 g/l (3 l). 20 ml de cette solution concentrée sont congelés et lyophilisés pour les analyses. Les degrés de substitution en MC et en B sont respectivement 0,79 ± 0,07 et 0,22 ± 0,03.

Sulfatation

La sulfatation nécessite la préparation du sel de triéthylammonium de DCMB.

La solution obtenue précédemment est éluée à travers une colonne de résine échangeuse de cation IR 120 H+ (1,5 l). La solution ainsi acidifiée est neutralisée avec de la triéthylamine jusqu'à un pH proche de 7,0.

La solution neutralisée est concentrée et lyophilisée. Environ 65 g de sel de triéthylammonium sont obtenus.

Les 65 g (0,39 mole de OH libre) de sel obtenus sont séchés dans une étuve sous vide à 40°C pendant 5 h ainsi que tout le matériel nécessaire. Puis on les dissout, dans un ballon tricol muni d'un système d'agitation et de circulation d'argon, dans 1,7 l de DMSO préalablement séché sur des tamis moléculaires 4 Å. 24,5 g (0,154 mole) (rapport molaire complexe/OH libre=0,4) de complexe $SO_3$-pyridine sont dissous dans 300 ml de DMSO et ajoutés lentement à la solution de sel de triéthylammonium de DCMB. L'ensemble est laissé sous agitation pendant 2 h à la température ambiante et sous argon.

La réaction est arrêtée en ajoutant 2 l d'eau bidistillée à 4°C au mélange et on ramène le pH du milieu à 7,5-8 à l'aide d'une solution de NaOH 2M. La solution est ensuite diluée jusqu'à obtenir une concentration en DMSO de 0,5 % en volume et ultrafiltrée à volume constant avec de l'eau bidistillée sur une membrane de seuil de coupure 5 000 g/mole. La solution est alors concentrée jusqu'à un volume de 5l et ultrafiltrée à volume constant sur la même membrane successivement avec 10 l de tampon carbonate 0,05 M pH 9,6 et avec de l'eau bidistillée jusqu'à une conductivité du filtrat satisfaisante. La solution ainsi purifiée est concentrée au septième de son volume, congelée et lyophilisée. 60 g de DCMBSul sont ainsi obtenus. L'analyse élémentaire du soufre donne un ds en groupements sulfates de $0,15 \pm 0,02$.

b) DCMBSu2

Carboxyméthylation

Dans un réacteur double enveloppe de 2 l maintenu à température ambiante et muni d'un système d'agitation, on disperse 50 g (0,31 mole) de dextrane T40 de masse molaire de 40 000 environ dans 612 ml d'isopropanol.

Dans un bécher de 500 ml, on dissout 67,9 g (1,7 moles) de NaOH dans 188 ml. La solution obtenue est refroidie à 4°C et ajoutée lentement au mélange dextrane-isopropanol. L'ensemble est laissé sous agitation pendant 1 heure.

Dans un bécher de 1 l, on dissout 87,5 g (0,93 mole) d'acide monochloroacétique dans 450 ml d'isopropanol (rapport molaire acide/OH=3).

La solution d'acide monochloroacétique est ajoutée rapidement dans le réacteur double enveloppe et l'ensemble est porté à 60°C à l'aide d'un thermostat relié au réacteur. L'ensemble est maintenu à cette température, sous agitation, pendant 2 h.

La phase aqueuse est ensuite récupérée et diluée jusqu'à obtenir un volume de 2,5 l. La solution ainsi obtenue est purifiée à volume constant sur une membrane de seuil de coupure 5 000 g/mole, avec de l'eau bidistillée jusqu'à obtenir une conductivité du filtrat satisfaisante. La concentration de la solution est de 28 g/l. 20 ml de cette solution sont congelés et lyophilisés pour les analyses.

On obtient 70 g (0,28 mole) de DCM avec un degré de substitution en CM de $1,1 \pm 0,1$.

Benzylamidificalion

Le pH de la solution précédente est ajusté à 4,75. Le mélange est refroidi à 4°C. On y ajoute rapidement 85,7 g d'agent de couplage (CMC) (rapport molaire CMC/CM=0,7). Dès que l'agent de couplage est entièrement solubilisé dans le mélange, on ajoute rapidement 22,2 ml de benzylamine (rapport molaire benzylamine/CM=0,7). Le mélange est ensuite laissé sous agitation pendant 16 heures à la température ambiante.

Le mélange est ensuite ultrafiltré à volume constant sur une membrane de seuil de coupure 5 000 g/mole, successivement avec 15 l 1 d'eau osmosée jusqu'à pH 7, puis avec 10 l de tampon carbonate 0,05 M pH 9,6, et enfin avec de l'eau bidistillée jusqu'à une conductivité du filtrat satisfaisante. La concentration de cette solution est de 21 g/l (3 l). 20 ml de cette solution concentrée sont congelés et lyophilisés pour les analyses. Les degrés de substitution en CM et en B sont respectivement $0,85 \pm 0,07$ et $0,27 \pm 0,03$.

Sulfatation

La solution obtenue précédemment est éluée à travers une colonne de résine échangeuse de cation IR 120 H+ (1,5 l). La solution ainsi acidifiée est neutralisée avec de la triéthylamine jusqu'à un pH proche de 7.

La solution neutralisée est concentrée et lyophilisée. Environ 65 g de sel de triéthylammonium sont obtenus.

Les 65 g (0,365 mole de OH libre) de sel obtenus sont séchés dans une étuve sous vide à 40°C pendant 5 h ainsi que tout le matériel nécessaire. Puis on les dissout, dans un ballon tricol muni d'un système d'agitation et de circulation d'argon, dans 1,7 l de DMSO préalablement séché sur des tamis moléculaires 4 Å. 22,9 g (0,144 mole) (rapport molaire complexe/OH libre=0,4) de complexe $SO_3$-pyridine sont dissous dans 300 ml de DMSO et ajoutés lentement à la solution de polymère. L'ensemble est laissé sous agitation pendant 2 h à la température ambiante et sous argon.

La réaction est arrêtée en ajoutant 2 l d'eau bidistillée à 4°C au mélange et on ramène le pH du milieu à 7,5-8 à l'aide d'une solution de NaOH 2M. La solution est ensuite diluée jusqu'à obtenir une concentration en DMSO de 0,5 % en volume et ultrafiltrée à volume constant avec de l'eau bidistillée sur une membrane de seuil de coupure 5 000 g/moie. La solution est alors concentrée jusqu'à un volume de 5 l et ultrafiltrée à volume constant sur la même membrane successivement avec 10 1 de tampon carbonate 0,05 M pH 9,6 et avec de l'eau bidistillée jusqu'à une conductivité du filtrat satisfaisante. La solution ainsi purifiée est concentrée au septième de son volume, congelée et lyophilisée. 60 g de DCMBSu2 sont ainsi obtenus avec un degré de substitution en groupements Su de $0,15 \pm 0,02$.

c) <u>DCMBSu3</u>

<u>Carboxyméthylation</u>

Dans un réacteur double enveloppe de 2 l maintenu à température ambiante et muni d'un système d'agitation, on disperse 50 g (0,31 mole) de dextrane T40 de masse molaire de 40 000 environ dans 612 ml d'isopropanol.

Dans un bécher de 500 ml, on dissout 67,9 g (1,7 moles) de NaOH dans 188 ml. La solution obtenue est refroidie à 4°C et ajoutée lentement au mélange dexirane-isopropanol. L'ensemble est laissé sous agitation pendant 1 heure.

Dans un bécher de 1 l, on dissout 72,9 g (0,77 mole) d'acide monochloroacétique dans 450 ml d'isopropanol (rapport molaire acide/OH=2,5).

La solution d'acide monochloroacétique est ajoutée rapidement dans le réacteur double enveloppe et l'ensemble est porté à 60°C à l'aide d'un thermostat relié au réacteur. L'ensemble est maintenu à cette température, sous agitation, pendant 2 h.

La phase aqueuse est ensuite récupérée et purifiée :

- soit par une double précipitation dans 3 l de méthanol. Le précipité ainsi récupéré est lavé deux fois au méthanol et séché dans une étuve sous vide à 40°C,
- soit par filtration tangentielle sur une membrane de seuil de coupure de 5 000 daltons.

La solution purifiée est alors concentrée et lyophilisée. On obtient 70 g (0,289 mole) de DCM avec un degré de substitution en CM de $1,0 \pm 0,1$.

<u>Recarboxyméthylation du DCM</u>

Dans un réacteur double enveloppe de 3 l maintenu à température ambiante et muni d'un système d'agitation, on disperse les 70 g (0,289 mole ou 0,578 mole de OH libre) de DCM obtenus dans 1035 ml d'isopropanol.

Dans un bécher de 500 ml, on dissout 46,4 g (1,16 moles) de NaOH dans 265 ml. La solution obtenue est refroidie à 4°C et ajoutée lentement au mélange dextrane-isopropanol. L'ensemble est laissé sous agitation pendant 1 heure.

Dans un bécher de 1 l, on dissout 54,6 g (0,578 mole) d'acide monochloroacétique dans 450 ml d'isopropanol (rapport molaire acide/OH libre=1).

La solution d'acide monochloroacétique est ajoutée rapidement dans le réacteur double enveloppe et l'ensemble est porté à 60°C à l'aide d'un thermostat relié au réacteur. L'ensemble est maintenu à cette température, sous agitation, pendant 2 h.

La phase aqueuse est ensuite récupérée et diluée jusqu'à obtenir un volume de 2,5 l. La solution ainsi obtenue est purifiée à volume constant sur une membrane de seuil de coupure 5 000 g/mole, avec de l'eau bidistillée jusqu'à obtenir une conductivité du filtrat satisfaisante. La concentration de la solution est de 30 g/l. 20 ml de cette solution sont congelés et lyophilisés pour les analyses.

On obtient 75 g (0,26 mole) de DCM avec un degré de substitution en CM de $1,58 \pm 0,12$.

<u>Benzylamidification</u>

Le pH de la solution précédente est ajusté à 4,75. On y ajoute rapidement 169 g d'agent de couplage (CMC) (rapport molaire CMC/CM=1). Dès que l'agent de couplage est entièrement solubilisé dans le mélange, on ajoute rapidement 43,7 ml de benzylamine (rapport molaire benzylamine/CM=1). Le mélange est ensuite laissé sous agitation pendant 16 heures à la température ambiante.

Le mélange est ensuite ultrafiltré à volume constant sur une membrane de seuil de coupure 5 000 g/mole, successivement avec 15 1 d'eau osmosée jusqu'à pH 7, puis avec 10 1 de tampon carbonate 0,05 M pH 9,6, et enfin avec de l'eau bidistillée jusqu'à une conductivité du filtrat satisfaisante. La concentration de cette solution est de 22 g/l (3 l). 20 ml de cette solution sont congelés et lyophilisés pour les analyses. Les degrés de substitution en CM et en B sont respectivement $1,15 \pm 0,10$ et $0,44 \pm 0,04$.

Sulfatation

La solution obtenue précédemment est éluée à travers une colonne de résine échangeuse de cation IR 120 H+ (1,5 l). La solution ainsi acidifiée est neutralisée avec de la triéthylamine jusqu'à un pH proche de 7.

La solution neutralisée est concentrée et lyophilisée. Environ 70 g de sel de triéthylammonium sont obtenus.

Les 70 g (0,24 mole de OH libre) de sel obtenus sont séchés dans une étuve sous vide à 40°C pendant 5 h ainsi que tout le matériel nécessaire. Puis on les dissout, dans un ballon tricol muni d'un système d'agitation et de circulation d'argon, dans 1,7 l de DMSO préalablement séché sur des tamis moléculaires 4 Å. 30,7 g (0,193 mole) (rapport molaire complexe/OH libre=0,8) de complexe $SO_3$-pyridine sont dissous dans 300 ml de DMSO et ajoutés lentement à la solution de polymère. L'ensemble est laissé sous agitation pendant 2 h à la température ambiante et sous argon.

La réaction est arrêtée en ajoutant 2 l d'eau bidistillée à 4°C au mélange et on ramène le pH du milieu à 7,5-8 à l'aide d'une solution de NaOH 2M. La solution est ensuite diluée jusqu'à obtenir une concentration en DMSO de 0,5 % en volume et ultrafiltrée à volume constant avec de l'eau bidistillée sur une membrane de seuil de coupure 5 000 g/mole. La solution est alors concentrée jusqu'à un volume de 5 l et ultrafiltrée à volume constant sur la même membrane successivement avec 10 l de tampon carbonate 0,05 M pH 9,6 et avec de l'eau bidistillée jusqu'à une conductivité du filtrat satisfaisante. La solution ainsi purifiée est concentrée au septième de son volume, congelée et lyophilisée. 65 g de DCMBSu3 sont ainsi obtenus avec un degré de substitution en groupements sulfates de $0,40 \pm 0,04$.

d) DCMBSu4

Carboxyméthylation

Dans un réacteur double enveloppe de 2 l maintenu à température ambiante et muni d'un système d'agitation, on disperse 50 g (0,31 mole) de dextrane T40 de masse molaire de 40 000 environ dans 612 ml d'isopropanol.

Dans un bécher de 500 ml, on dissout 67,9 g (1,7 moles) de NaOH dans 188 ml. La solution obtenue est refroidie à 4°C et ajoutée lentement au mélange dextrane-isopropanol. L'ensemble est laissé sous agitation pendant 1 heure.

Dans un bécher de 1 l, on dissout 72,9 g (0,77 mole) d'acide monochloroacétique dans 450 ml d'isopropanol (rapport molaire acide/OH=2,5).

La solution d'acide monochloroacétique est ajoutée rapidement dans le réacteur double enveloppe et l'ensemble est porté à 60°C à l'aide d'un thermostat relié au réacteur. L'ensemble est maintenu à cette température, sous agitation, pendant 2 h.

La phase aqueuse est ensuite récupérée et diluée jusqu'à obtenir un volume de 2,5 l. La solution ainsi obtenue est purifiée à volume constant sur une membrane de seuil de coupure 5 000 g/mole, avec de l'eau bidistillée jusqu'à obtenir une conductivité du filtrat satisfaisante. La concentration de la solution est de 28 g/l. 20 ml de cette solution sont congelés et lyophilisés pour les analyses.

On obtient 70 g (0,284 mole) de DCM avec un degré de substitution en CM de $1,05 \pm 0,10$.

Benzylamidification

Le pH de la solution précédente est ajusté à 4,75. On y ajoute rapidement 126,5 g d'agent de couplage (CMC) (rapport molaire CMC/CM=0,8). Dès que l'agent de couplage est entièrement solubilisé dans le mélange, on ajoute rapidement 32,6 ml de benzylamine (rapport molaire benzylamine/CM=0,8). Le mélange est ensuite laissé sous agitation pendant 16 heures à la température ambiante.

Le mélange est ensuite ultrafiltré à volume constant sur une membrane de seuil de coupure 5 000 g/mole, successivement avec 15 1 d'eau osmosée jusqu'à pH 7, puis avec 10 l de tampon carbonate 0,05 M pH 9,6, et enfin avec de l'eau bidistillée jusqu'à une conductivité du filtrat satisfaisante. La concentration de cette solution est de 21 g/l (3 l). 20 ml de cette solution sont congelés et lyophilisés pour les analyses. Les degrés de substitution en CM et en B sont respectivement $0,74 \pm 0,06$ et $0,33 \pm 0,03$.

Sulfatation

La solution obtenue précédemment est éluée à travers une colonne de résine échangeuse de cation IR 120 H+ (1,5 l). La solution ainsi acidifiée est neutralisée avec de la triéthylamine jusqu'à un pH proche de 7,0.

La solution neutralisée est concentrée et lyophilisée. Environ 65 g de sel de triéthylammonium sont obtenus.

Les 65 g (0,38 mole de OH libre) de sel obtenus sont séchés dans une étuve sous vide à 40°C pendant 5 h ainsi que tout le matériel nécessaire. Puis on les dissout, dans un ballon tricol muni d'un système d'agitation et de circulation d'argon, dans 1,7 l de DMSO préalablement séché sur des tamis moléculaires 4 Å. 30,2 g (0,154 mole) (rapport molaire complexe/OH libre=0,5) de complexe $SO_3$-pyridine sont dissous dans 300 ml de DMSO et ajoutés lentement à la solution de polymère. L'ensemble est laissé sous agitation pendant 2 h à la température ambiante et sous argon.

La réaction est arrêtée en ajoutant 2 1 d'eau bidistillée à 4°C au mélange et on ramène le pH du milieu à 7,5-8 à l'aide d'une solution de NaOH 2M. La solution est ensuite diluée jusqu'à obtenir une concentration en DMSO de 0,5 % en volume et ultrafiltrée à volume constant avec de l'eau bidistillée sur une membrane de seuil de coupure 5 000 g/mole. La solution est alors concentrée jusqu'à un volume de 5 l et ultrafiltrée à volume constant sur la même membrane successivement avec 10 l de tampon carbonate 0,05 M pH 9,6 et avec de l'eau bidistillée jusqu'à une conductivité du filtrat satisfaisante. La solution ainsi purifiée est concentrée au septième de son volume, congelée et lyophilisée. 60 g de DCMBSu4 sont ainsi obtenus avec un degré de substitution en groupements Su de $0,25 \pm 0,03$.

e) DCMBSu5

Carboxyméthylation

Dans un réacteur double enveloppe de 2 1 maintenu à température ambiante et muni d'un système d'agitation, on disperse 50 g (0,31 mole) de dextrane T40 de masse molaire de 40 000 environ dans 612 ml d'isopropanol.

Dans un bécher de 500 ml, on dissout 67,9 g (1,7 moles) de NaOH dans 188 ml. La solution obtenue est refroidie à 4°C et ajoutée lentement au mélange dextrane-isopropanol. L'ensemble est laissé sous agitation pendant 1 heure.

Dans un bécher de 1 l, on dissout 117,2 g (1,24 mole) d'acide monochloroacétique dans 450 ml d'isopropanol (rapport molaire acide/OH=4).

La solution d'acide monochloroacétique est ajoutée rapidement dans le réacteur double enveloppe et l'ensemble est porté à 60°C à l'aide d'un thermostat relié au réacteur. L'ensemble est maintenu à cette température, sous agitation, pendant 2 h.

La phase aqueuse est ensuite récupérée et diluée jusqu'à obtenir un volume de 2,5 l. La solution ainsi obtenue est purifiée à volume constant sur une membrane de seuil de coupure 5 000 g/mole, avec de l'eau bidistillée jusqu'à obtenir une conductivité du filtrat satisfaisante. La concentration de la solution est de 28 g/l. 20 ml de cette solution sont congelés et lyophilisés pour les analyses.

On obtient 70 g (0,271 mole) de DCM avec un degré de substitution en CM de $1,2 \pm 0,11$.

Benzylamidification

Le pH de la solution précédente est ajusté à 4,75. On y ajoute rapidement 124,1 g d'agent de couplage (CMC) (rapport molaire CMC/CM=0,9). Dès que l'agent de couplage est entièrement solubilisé dans le mélange, on ajoute rapidement 35,5 ml de benzylamine (rapport molaire benzylamine/CM=0,9). Le mélange est ensuite laissé sous agitation pendant 16 heures à la température ambiante.

Le mélange est ensuite ultrafiltré à volume constant sur une membrane de seuil de coupure 5 000 g/mole, successivement avec 15 l d'eau osmosée jusqu'à pH 7, puis avec 10 l de tampon carbonate 0,05 M pH 9,6, et enfin avec de l'eau bidistillée jusqu'à une conductivité du filtrat satisfaisante. La concentration de cette solution est de 21 g/l (3 l). 20 ml de cette solution sont congelés et lyophilisés pour les analyses. Les degrés de substitution en CM et en B sont respectivement $0,84 \pm 0,07$ et $0,38 \pm 0,04$.

Sulfatation

La solution obtenue précédemment est éluée à travers une colonne de résine échangeuse de cation IR 120 H+ (1,5 l). La solution ainsi acidifiée est neutralisée avec de la triéthylamine jusqu'à un pH proche de 7.

La solution neutralisée est concentrée et lyophilisée. Environ 67 g de sel de triéthylammonium sont obtenus.

Les 67 g (0,33 mole de OH libre) de sel obtenus sont séchés dans une étuve sous vide à 40°C pendant 5 h ainsi que tout le matériel nécessaire. Puis on les dissout, dans un ballon tricol muni d'un système d'agitation et de circulation d'argon, dans 1,7 l de DMSO préalablement séché sur des tamis moléculaires 4 Å. 21 g (0,132 mole) (rapport molaire complexe/OH libre=0,4) de complexe $SO_3$-pyridine sont dissous dans 300 ml de DMSO et ajoutés lentement à la solution de polymère. L'ensemble est laissé sous agitation pendant 2 h à la température ambiante et sous argon.

La réaction est arrêtée en ajoutant 2 l d'eau bidistillée à 4°C au mélange et on ramène le pH du milieu à 7,5-8 à l'aide d'une solution de NaOH 2M. La solution est ensuite diluée jusqu'à obtenir une concentration en DMSO de 0,5 % en volume et ultrafiltrée à volume constant avec de l'eau bidistillée sur une membrane de seuil de coupure 5 000 g/mole. La solution est alors concentrée jusqu'à un volume de 5 l et ultrafiltrée à volume constant sur la même membrane successivement avec 10 1 de tampon carbonate 0,05 M pH 9,6 et avec de l'eau bidistillée jusqu'à une conductivité du filtrat satisfaisante. La solution ainsi purifiée est concentrée au septième de son volume, congelée et lyophilisée. 62 g de DCMBSu5 sont ainsi obtenus avec un degré de substitution en groupements sulfates de $0,15 \pm 0,02$.

f) DCMBSu6

Carboxyméthylation

Dans un réacteur double enveloppe de 2 l maintenu à température ambiante et muni d'un système d'agitation, on disperse 50 g (0,31 mole) de dextrane T40 de masse molaire de 40 000 environ dans 612 ml d'isopropanol.

Dans un bécher de 500 ml, on dissout 67,9 g (1,7 moles) de NaOH dans 188 ml. La solution obtenue est refroidie à 4°C et ajoutée lentement au mélange dextrane-isopropanol. L'ensemble est laissé sous agitation pendant 1 heure.

Dans un bécher de 1 l, on dissout 87,5 g (0,93 mole) d'acide monochloroacétique dans 450 ml d'isopropanol (rapport molaire acide/OH=3).

La solution d'acide monochloroacétique est ajoutée rapidement dans le réacteur double enveloppe et l'ensemble est porté à 60°C à l'aide d'un thermostat relié au réacteur. L'ensemble est maintenu à cette température, sous agitation, pendant 2 h.

La phase aqueuse est ensuite récupérée et diluée jusqu'à obtenir un volume de 2,5 l. La solution ainsi obtenue est purifiée à volume constant sur une membrane de seuil de coupure 5 000 g/mole, avec de l'eau bidistillée jusqu'à obtenir une conductivité du filtrat satisfaisante. La concentration de la solution est de 28 g/l. 20 ml de cette solution sont congelés et lyophilisés pour les analyses.

On obtient 70 g (0,28 mole) de DCM avec un degré de substitution en CM de $1,1 \pm 0,10$.

1ère benzylamidification

Le pH de la solution précédente est ajusté à 4,75. On y ajoute rapidement 130,5 g d'agent de couplage (CMC) (rapport molaire CMC/CM=1). Dès que l'agent de couplage est entièrement solubilisé dans le mélange, on ajoute rapidement 30,3 ml de benzylamine (rapport molaire benzylamine/CM=0,9). Le mélange est ensuite laissé sous agitation pendant 16 heures à la température ambiante.

Le mélange est ensuite ultrafiltré à volume constant sur une membrane de seuil de coupure 5 000 g/mole, successivement avec 15 l d'eau osmosée jusqu'à pH 7, puis avec 10 l de tampon carbonate 0,05 M pH 9,6, et enfin avec de l'eau bidistillée jusqu'à une conductivité du filtrat satisfaisante. La solution est concentrée jusqu'à un volume de 2,5 l (~28 g/l ou 0,1 mole/l). 20 ml de cette solution sont congelés et lyophilisés pour les analyses. Les degrés de substitution en CM et en B sont respectivement $0,75 \pm 0,06$ et $0,38 \pm 0,04$.

2ème benzylamidification

Le pH des 2,5 1 de solution de DCMB précédente est réajusté à 4,75. Le mélange est refroidi à 4°C. On y ajoute rapidement 40 g d'agent de couplage (CMC) (rapport molaire CMC/CM=0,5). Dès que l'agent de couplage est entièrement solubilisé dans le mélange, on ajoute rapidement 10,3 ml de benzylamine (rapport molaire benzylamine/CM=0,5). Le mélange est ensuite laissé sous agitation pendant 16 heures à la température ambiante.

Le mélange est ensuite ultrafiltré à volume constant sur une membrane de seuil de coupure 5 000 g/mole, successivement avec 15 l d'eau osmosée jusqu'à pH 7, puis avec 10 l de tampon carbonate 0,05 M pH 9,6, et enfin avec de l'eau bidistillée jusqu'à une conductivité du filtrat satisfaisante. La concentration de cette solution est de 21 g/l (3 l). 20 ml de cette solution sont congelés et lyophilisés pour les analyses. Les degrés de substitution en CM et en B sont respectivement $0,65 \pm 0,06$ et $0,54 \pm 0,05$.

Sulfatation

La solution obtenue précédemment est éluée à travers une colonne de résine échangeuse de cation 1R 120 H+ (1,5 l). La solution ainsi acidifiée est neutralisée avec de la triéthylamine jusqu'à un pH proche de 7.

La solution neutralisée est concentrée et lyophilisée. Environ 65 g de sel de triéthylammonium sont obtenus.

Les 65 g (0,365 mole de OH libre) de sel obtenus sont séchés dans une étuve sous vide à 40°C pendant 5 h ainsi que tout le matériel nécessaire. Puis on les dissout, dans un ballon tricol muni d'un système d'agitation et de circulation d'argon, dans 1,7 l de DMSO préalablement séché sur des tamis moléculaires 4 Å. 34,9 g (0,219 mole) (rapport molaire complexe/OH libre=0,6) de complexe $SO_3$-pyridine sont dissous dans 300 ml de DMSO et ajoutés lentement à la solution de polymère. L'ensemble est laissé sous agitation pendant 2 h à la température ambiante et sous argon.

La réaction est arrêtée en ajoutant 2 l d'eau bidistillée à 4°C au mélange et on ramène le pH du milieu à 7,5-8 à l'aide d'une solution de NaOH 2M. La solution est ensuite diluée jusqu'à obtenir une concentration en DMSO de 0,5 % en volume et ultrafiltrée à volume constant avec de l'eau bidistillée sur une membrane de seuil de coupure 5 000 g/mole. La solution est alors concentrée jusqu'à un volume de 5 l et ultrafiltrée à volume constant sur la même membrane successivement avec 10 l de tampon carbonate 0,05 M pH 9,6 et avec de l'eau bidistillée jusqu'à une conductivité du filtrat satisfaisante. La solution ainsi purifiée est concentrée au septième de son volume, congelée et lyophilisée. 60 g de DCMBSu6 sont ainsi obtenus avec un degré de substitution en groupements sulfates de 0,30 ± 0,03.

g) DCMBSu7

Carboxyméthylation

Dans un réacteur double enveloppe de 2 l maintenu à température ambiante et muni d'un système d'agitation, on disperse 50 g (0,31 mole) de dextrane T40 de masse molaire de 40 000 environ dans 612 ml d'isopropanol.

Dans un bécher de 500 ml, on dissout 67,9 g (1,7 moles) de NaOH dans 188 ml. La solution obtenue est refroidie à 4°C et ajoutée lentement au mélange dextrane-isopropanol. L'ensemble est laissé sous agitation pendant 1 heure.

Dans un bécher de 1 l, on dissout 72,9 g (0,77 mole) d'acide monochloroacétique dans 450 ml d'isopropanol (rapport molaire acide/OH=2,5).

La solution d'acide monochloroacétique est ajoutée rapidement dans le réacteur double enveloppe et l'ensemble est porté à 60°C à l'aide d'un thermostat relié au réacteur. L'ensemble est maintenu à cette température, sous agitation, pendant 2 h.

La phase aqueuse est ensuite récupérée et diluée jusqu'à obtenir un volume de 2,5 l. La solution ainsi obtenue est purifiée à volume constant sur une membrane de seuil de coupure 5 000 g/mole, avec de l'eau bidistillée jusqu'à obtenir une conductivité du filtrat satisfaisante. La concentration de la solution est de 28 g/l. 20 ml de cette solution sont congelés et lyophilisés pour les analyses.

On obtient 70 g (0,284 mole) de DCM avec un degré de substitution en CM de 1,05.

Benzylamidification

Le pH de la solution précédente est ajusté à 4,75. On y ajoute rapidement 126,5 g d'agent de couplage (CMC) (rapport molaire CMC/CM=0,8). Dès que l'agent de couplage est entièrement solubilisé dans le mélange, on ajoute rapidement 32,6 ml de benzylamine (rapport molaire benzylamine/CM=0,8). Le mélange est ensuite laissé sous agitation pendant 16 heures à la température ambiante.

Le mélange est ensuite ultrafiltré à volume constant sur une membrane de seuil de coupure 5 000 g/mole, successivement avec 15 l d'eau osmosée jusqu'à pH 7, puis avec 10 l de tampon carbonate 0,05 M pH 9,6, et enfin avec de l'eau bidistillée jusqu'à une conductivité du filtrat satisfaisante. La concentration de cette solution est de 21 g/l (3 l). 20 ml de cette solution sont congelés et lyophilisés pour les analyses. Les degrés de substitution en CM et en B sont respectivement 0,74 ± 0,06 et 0,33 ± 0,03.

Sulfatation

La solution obtenue précédemment est éluée à travers une colonne de résine échangeuse de cation IR 120 H+ (1,5 l). La solution ainsi acidifiée est neutralisée avec de la triéthylamine jusqu'à un pH proche de 7.

La solution neutralisée est concentrée et lyophilisée. Environ 65 g de sel de triéthylammonium sont obtenus.

Les 65 g (0,38 mole de OH libre) de sel obtenus sont séchés dans une étuve sous vide à 40°C pendant 5 h ainsi que tout le matériel nécessaire. Puis on les dissout, dans un ballon tricol muni d'un système d'agitation et de circulation d'argon, dans 1,7 l de DMSO préalablement séché sur des tamis moléculaires 4 Å. 24,2 g (0,154 mole) (rapport molaire complexe/OH libre=0,4) de complexe SO$_3$-pyridine sont dissous dans 300 ml de DMSO et ajoutés lentement à la solution de polymère. L'ensemble est laissé sous agitation pendant 2 h à la température ambiante et sous argon.

La réaction est arrêtée en ajoutant 2 l d'eau bidistillée à 4°C au mélange et on ramène le pH du milieu à 7,5-8 à l'aide d'une solution de NaOH 2M. La solution est ensuite diluée jusqu'à obtenir une concentration en DMSO de 0,5 % en volume et ultrafiltrée à volume constant avec de l'eau bidislillée sur une membrane de seuil de coupure 5 000 g/mole. La solution est alors concentrée jusqu'à un volume de 5 l et ultrafiltrée à volume constant sur la même membrane successivement avec 10 l de tampon carbonate 0,05 M pH 9,6 et avec de l'eau bidistillée jusqu'à une conductivité du filtrat satisfaisante. La solution ainsi purifiée est concentrée au septième de son volume, congelée et lyophilisée. 60 g de DCMBSu7 sont ainsi obtenus avec un degré de substitution en groupements sulfates de 0,16 ± 0,02.

h) DCMBSu8

Carboxyméthylation

Dans un réacteur double enveloppe de 2 l maintenu à température ambiante et muni d'un système d'agitation, on disperse 50 g (0,31 mole) de dextrane T40 de masse molaire de 40 000 environ dans 612 ml d'isopropanol.

Dans un bécher de 500 ml, on dissout 67,9 g (1,7 moles) de NaOH dans 188 ml. La solution obtenue est refroidie à 4°C et ajoutée lentement au mélange dextrane-isopropanol. L'ensemble est laissé sous agitation pendant 1 heure.

Dans un bécher de 1 l, on dissout 72,9 g (0,77 mole) d'acide monochloroacétique dans 450 ml d'isopropanol (rapport molaire acide/OH=2,5).

La solution d'acide monochloroacétique est ajoutée rapidement dans le réacteur double enveloppe et l'ensemble est porté à 60°C à l'aide d'un thermostat relié au réacteur. L'ensemble est maintenu à cette température, sous agitation, pendant 1 h.

La phase aqueuse est ensuite récupérée et diluée jusqu'à obtenir un volume de 2,5 l. La solution ainsi obtenue est purifiée à volume constant sur une membrane de seuil de coupure 5 000 g/mole, avec de l'eau bidistillée jusqu'à obtenir une conductivité du filtrat satisfaisante. La concentration de la solution est de 28 g/l. 20 ml de cette solution sont congelés et lyophilisés pour les analyses.

On obtient 70 g (0,295 mole) de DCM avec un degré de substitution en CM de 0,94 ± 0,10.

Benzylamidification

Le pH de la solution précédente est ajusté à 4,75. Le mélange est refroidi à 4°C. On y ajoute rapidement 58,8 g d'agent de couplage (CMC) (rapport molaire CMC/CM=0,5). Dès que l'agent de couplage est entièrement solubilisé dans le mélange, on ajoute rapidement 15,2 ml de benzylamine (rapport molaire benzylamine/CM=0,5). Le mélange est ensuite laissé sous agitation pendant 16 heures à la température ambiante.

Le mélange est ensuite ultrafiltré à volume constant sur une membrane de seuil de coupure 5 000 g/mole, successivement avec 15 l d'eau osmosée jusqu'à pH 7, puis avec 10 l de tampon carbonate 0,05 M pH 9,6, et enfin avec de l'eau bidistillée jusqu'à une conductivité du filtrat satisfaisante. La concentration de cette solution est de 21 g/l (3 l). 5 ml de cette solution concentrée sont congelés et lyophilisés pour les analyses. Les degrés de substitution en CM et en B sont respectivement 0,75 ± 0,07 et 0,22 ± 0,03.

Sulfatation

La solution obtenue précédemment est éluée à travers une colonne de résine échangeuse de cation IR 120 H+ (1,5 l). La solution ainsi acidifiée est neutralisée avec de la triéthylamine jusqu'à un pH proche de 7.

La solution neutralisée est concentrée et lyophilisée. Environ 65 g de sel de triéthylammonium sont obtenus.

Les 65 g (0,4 mole de OH libre) de sel obtenus sont séchés dans une étuve sous vide à 40°C pendant 5 h ainsi que tout le matériel nécessaire. Puis on les dissout, dans un ballon tricol muni d'un système d'agitation et de circulation d'argon, dans 1,7 l de DMSO préalablement séché sur des tamis moléculaires 4 Å. 45,2 g (0,154 mole) (rapport molaire complexe/OH libre=0,7) de complexe SO$_3$-pyridine sont dissous dans 300 ml de DMSO et ajoutés lentement à la solution de polymère. L'ensemble est laissé sous agitation pendant 2 h à la température ambiante et sous argon.

La réaction est arrêtée en ajoutant 2 l d'eau bidistillée à 4°C au mélange et on ramène le pH du milieu à 7,5-8 à l'aide d'une solution de NaOH 2M. La solution est ensuite diluée jusqu'à obtenir une concentration en DMSO de 0,5 % en volume et ultrafiltrée à volume constant avec de l'eau bidistillée sur une membrane de seuil de coupure 5 000

EP 0 990 002 B1

g/mole. La solution est alors concentrée jusqu'à un volume de 5 l et ultrafiltrée à volume constant sur la même membrane successivement avec 10 l de tampon carbonate 0,05 M pH 9,6 et avec de l'eau bidistillée jusqu'à une conductivité du filtrat satisfaisante. La solution ainsi purifiée est concentrée au septième de son volume, congelée et lyophilisée. 60 g de DCMBSu8 sont ainsi obtenus avec un degré de substitution en groupements sulfates de $0,35 \pm 0,04$.

i) <u>DCMSu</u>

<u>Carboxyméthylation</u>

Dans un réacteur double enveloppe de 2 l maintenu à température ambiante et muni d'un système d'agitation, on disperse 50 g (0,31 mole) de dextrane T40 de masse molaire de 40 000 environ dans 612 ml d'isopropanol.

Dans un bécher de 500 ml, on dissout 67,9 g (1,7 moles) de NaOH dans 188 ml. La solution obtenue est refroidie à 4°C et ajoutée lentement au mélange dextrane-isopropanol. L'ensemble est laissé sous agitation pendant 1 heure.

Dans un bêcher de 1 l, on dissout 72,9 g (0,77 mole) d'acide monochloroacétique dans 450 ml d'isopropanol (rapport molaire acide/OH=2,5).

La solution d'acide monochloroacétique est ajoutée rapidement dans le réacteur double enveloppe et l'ensemble est porté à 60°C à l'aide d'un thermostat relié au réacteur. L'ensemble est maintenu à cette température, sous agitation, pendant 2 h.

La phase aqueuse est ensuite récupérée et diluée jusqu'à obtenir un volume de 2,5 l. La solution ainsi obtenue est purifiée à volume constant sur une membrane de seuil de coupure 5 000 g/mole, avec de l'eau bidistillée jusqu'à obtenir une conductivité du filtrat satisfaisante. La concentration de la solution est de 28 g/l. 20 ml de cette solution sont congelés et lyophilisés pour les analyses.

On obtient 70 g (0,289 mole) de DCM avec un degré de substitution en CM de $1,0 \pm 0,1$.

<u>Sulfatation</u>

La solution obtenue précédemment est éluée à travers une colonne de résine échangeuse de cation IR 120 H+ (1,5 l). La solution ainsi acidifiée est neutralisée avec de la triéthylamine jusqu'à un pH proche de 7.

La solution neutralisée est concentrée et lyophilisée. Environ 80 g de sel de triéthylammonium sont obtenus.

Les 80 g (0,466 mole de OH libre) de sel obtenus sont séchés dans une étuve sous vide à 40°C pendant 5 h ainsi que tout le matériel nécessaire. Puis on les dissout, dans un ballon tricol muni d'un système d'agitation et de circulation d'argon, dans 1,7 l de DMSO préalablement séché sur des tamis moléculaires 4 Å. 52 g (0,326 mole) (rapport molaire complexe/OH libre=0,7) de complexe $SO_3$-pyridine sont dissous dans 300 ml de DMSO et ajoutés lentement à la solution de polymère. L'ensemble est laissé sous agitation pendant 2 h à la température ambiante et sous argon.

La réaction est arrêtée en ajoutant 2 l d'eau bidistillée à 4°C au mélange et on ramène le pH du milieu à 7,5-8 à l'aide d'une solution de NaOH 2M. La solution est ensuite diluée jusqu'à obtenir une concentration en DMSO de 0,5 % en volume et ultrafiltrée à volume constant avec de l'eau bidistillée sur une membrane de seuil de coupure 5 000 g/mole. La solution est alors concentrée jusqu'à un volume de 5 l et ultrafiltrée à volume constant sur la même membrane successivement avec 10 l de tampon carbonate 0,05 M pH 9,6 et avec de l'eau bidistillée jusqu'à une conductivité du filtrat satisfaisante. La solution ainsi purifiée est concentrée au septième de son volume, congelée et lyophilisée. 70 g de DCMSu sont ainsi obtenus. L'analyse élémentaire du soufre donne un ds en groupements sulfates de $0,37 \pm 0,04$.

<u>**EXEMPLE 3 :**</u> **Préparation d'un DCMBSuS.**

On prépare une suspension de 10 g du DCMBSu, obtenu conformément à l'exemple 2, dans 350 ml de dichlorométhane anhydre. On ajoute 0,62 ml d'acide chlorosulfonique dans 35 ml de dichlorométhane anhydre (rapport molaire acide chlorosulfonique/motifs B = 1). Le mélange est maintenu sous agitation et sous argon pendant 1 heure à température ambiante. Le produit est filtré sur fritté N°4, lavé successivement avec 200 ml de dichlorométhane, 200 ml de dichlorométhane/dioxanne 50:50 (v:v) et enfin avec du dioxanne pur.

Le DCMBSuS sous forme acide est aussitôt dissous dans 150 ml d'eau et le pH de la solution est ajusté à 9 avec NaOH 6 M et maintenu à cette valeur pendant 1 heure. La solution est alors neutralisée avec HCl 0,1 M, congelée et lyophilisée.

La quantité de sulfonates est établie par différence des taux de soufre des produits initial et final après désulfatation des deux produits, dans les conditions précisées à l'exemple 1.

**EXEMPLE 4 : Variante de procédé pour la préparation d'un DCMBSu.**

**a) Préparation du réactif N-méthyl phényl 2 chloroacétamide**

Dans un ballon tricol muni d'un système d'agitation et de circulation d'argon (atmosphère inerte), on mélange 109,4 ml (1 mole) de benzylamine avec 1 l d'éther préalablement séché sur des tamis moléculaires 4 Å.

On ajoute goutte à goutte, à l'aide d'une ampoule à brome, 79,7 ml (1 mole) de chlorure de chloroacétyle (durée d'introduction 30 minutes). Après ajout, le ballon est scellé et laissé sous agitation à température ambiante pendant 1 heure.

Le mélange est ensuite lavé, successivement avec 1,5 l de solution d'acide citrique 0,5 M (220 g), 1,5 l d'eau bidistillée à 4°C, 1,5 l de solution d'hydrogéno-carbonate de sodium 0,5 M (63 g) et 1,5 l de solution NaCl 1 M (87,8 g).

La phase éthérée est récupérée et évaporée à sec.

Le résidu sec est purifié par une double recristallisation dans l'eau :

- On dissout le résidu dans de l'eau bidistillée, (solution de 1-2% p/v) portée à ébullition. On filtre cette solution bouillante à l'aide d'un verre fritté N°4 préchauffé à 100°C. La solution filtrée est refroidie et conservée à 4°C pendant une nuit. Le réactif est ensuite récupéré sur du papier Wattman et séché dans une étuve sous vide à 50°C environ.

**b) Synthèse de DCMB**

Dans un réacteur double enveloppe de 2 l muni d'un système d'agitation, on disperse 50 g (0,31 mole) de dextrane T40 de masse molaire de 40.000 environ dans 400 ml d'isopropanol.

Dans un bécher de 500 ml, on dissout 56,5 g (1,41 mole) de NaOH dans 225 ml. La solution obtenue est refroidie à 4°C et ajoutée lentement au mélange dextrane-isopropanol. L'ensemble est laissé sous agitation pendant 1 heure.

Dans un bécher de 1 l, on dissout 84,9 g (0,46 mole) du N-méthyl-phényl-2-chloroacétamide obtenu en a), dans 675 ml d'isopropanol. Dans un autre bécher de 500 ml, on dissout 43,7 g (0,46 mole) d'acide monochloroacétique dans 200 ml d'isopropanol.

La solution de N-méthyl-phényl-2-chloroacétamide est ajoutée rapidement dans le réacteur double enveloppe et l'ensemble est porté à 60°C à l'aide d'un thermostat relié au réacteur. Environ 5 minutes plus tard, la solution d'acide monochloroacétique y est rapidement ajoutée et l'ensemble est maintenu à 60°C sous agitation pendant 2 heures.

La phase aqueuse est ensuite récupérée et purifiée :

- soit par une double précipitation dans 3 l de méthanol ; le précipité ainsi récupéré est lavé deux fois au méthanol et séché dans une étuve sous vide à 40°C.
- soit par filtration tangentielle sur une membrane de seuil de coupure de 5.000 Daltons ; la solution purifiée est alors concentrée et lyophilisée.
  La composition du DCM obtenu par cette méthode est la suivante : CM = 0,90 $\pm$ 0,09 et B = 0,30 $\pm$ 0,03.

**c) Synthèse du DCMBSu**

**- mise sous forme de sel de tributylammonium (ou triéthylammonium)**

50 g (0,18 mole) du DCMB obtenu à l'étape b) sont dissous dans 2 l d'eau bidistillée. La solution obtenue est éluée à travers une colonne de résine échangeuse de cations (Amberlite IR120 H+). Le produit ainsi acidifié est neutralisé avec de la tributylamine à 10 % dans l'éthanol (ou de la triéthylamine) jusqu'à un pH proche de 7.

La solution neutralisée est purifiée par filtration tangentielle sur une membrane de seuil de coupure 5.000 daltons, concentrée et lyophilisée. Environ 60 g de sel de tributylammonium (ou triéthylammonium) sont obtenus.

**- Sulfatation du DCMB de tributylammonium (ou triéthylammonium**

Les 60 g de sel obtenus sont séchés dans une étuve sous vide à 40°C pendant 4 heures ainsi que tout le matériel nécessaire. Puis on les dissout, dans un ballon tricol muni d'un système d'agitation et de circulation d'argon, dans 2,7 l de N,N-diméthylformamide (DMF) préalablement séché sur des tamis moléculaires 4 Å (5,7 l pour le sel de triéthylammonium). 38,7 g (0,24 mole) de complexe SO₃-pyridine (46,6 g (0,29 mole) dans le cas du sel de triéthylammonium) sont dissous dans 300 ml de DMF et ajoutés lentement à la solution de polymère. L'ensemble est laissé sous agitation pendant 2 heures à température ambiante.

La réaction est arrêtée en ajoutant 3 1 d'eau bidistillée à 4°C au mélange et on ramène le pH du milieu à 7,5-8 à l'aide d'une solution de NaOH 2 M. La solution est ensuite ultrafiltrée sur membrane de seuil de coupure 5.000 daltons, concentrée et lyophilisée.

## EXEMPLE 5 : Synthèse du DCMSu.

### a) Synthèse du DCM

Dans un réacteur double enveloppe de 2 l muni d'un système d'agitation, on disperse 50 g (0,31 mole) de dextrane T40 de masse molaire de 40.000 environ dans 612 ml d'isopropanol.

Dans un bécher de 500 ml, on dissout 67,9 g (1,7 mole) de NaOH dans 188 ml. La solution obtenue est refroidie à 4°C et ajoutée lentement au mélange dextrane-isopropanol. L'ensemble est laissé sous agitation pendant 1 heure.

Dans un bécher de 1 l, on dissout 72,9 g (0,77 mole) d'acide monochloroacétique dans 450 ml d'isopropanol.

La solution d'acide monochloroacétique est ajoutée rapidement dans le réacteur double enveloppe et l'ensemble est porté à 60°C à l'aide d'un thermostat relié au réacteur. L'ensemble est maintenu à cette température, sous agitation, pendant 2 heures.

La phase aqueuse est ensuite récupérée et purifiée :

- soit par une double précipitation dans 3 l de méthanol ; le précipité ainsi récupéré est lavé deux fois au méthanol et séché dans une étuve sous vide à 40°C,
- soit par filtration tangentielle sur une membrane de seuil de coupure de 5.000 Daltons; la solution purifiée est alors concentrée et lyophilisée.

### b) Synthèse du DCMSu

**- mise sous forme de sel de tributylammonium (ou triéthylammonium)**

50 g (0,18 mole) de DCM sont dissous dans 2 l d'eau bidistillée. La solution obtenue est éluée à travers une colonne de résine échangeuse de cations (Amberlite IR120 H+). Le produit ainsi acidifié est neutralisé avec de la tributylammonium à 10% dans l'éthanol (ou la triéthylamine) jusqu'à un pH proche de 7.

La solution neutralisée est purifiée par filtration tangentielle sur une membrane de seuil de coupure de 5.000 Daltons, concentrée et lyophilisée. Environ 60 g de sel de tributylammonium (ou triéthylammonium) sont obtenus.

**- sulfatation du DCM de tributylammonium (ou triéthylammonium)**

Les 60 g de sel obtenus sont séchés dans une étuve sous vide à 40°C pendant 4 heures ainsi que tout le matériel nécessaire. Puis on les dissout, dans un ballon tricol muni d'un système d'agitation et de circulation d'argon, dans 2,7 l de N,N-diméthylformamide (DMF) préalablement séché sur des tamis moléculaires 4 Å. 44,8 g (0,28 mole) de complexe $SO_3$-pyridine ou 55,8 g (0,35 mole) dans le cas du sel de triéthylammonium), sont dissous dans 300 ml de DMF et ajoutés lentement à la solution de polymère. L'ensemble est laissé sous agitation pendant 2 heures à température ambiante et sous argon.

La réaction est arrêtée en ajoutant 3 l d'eau bidistillée à 4°C au mélange et le pH du milieu à 7,5-8 est ramené à l'aide d'une solution de NaOH 2 M. La solution est ensuite ultrafiltrée sur membrane de seuil de coupure 5.000 daltons, concentrée et lyophilisée.

La composition d'un DCMSu obtenu est la suivante : CM = 1 ± 0,1, Su = 0,37 ± 0,04.

## EXEMPLE 6 : Activité anticoagulante et anticomplémentaire des produits selon l'invention.

### a) Mesure de l'activité anticoagulante

Le temps de céphaline activée est un test d'exploration sensible à tous les facteurs de la voie endogène (thrombine, V, VIII, IX, XI et XII) du système de la coagulation à l'exception du facteur III plaquettaire. Le réactif utilisé (APTT, Organon Teknika) contient des phospholipides de cerveau de lapin (facteur III plaquettaire) et un activateur particulaire (silice micronisée ou acide ellagique). Le protocole utilisé est le suivant :

    100 µl de plasma humain pauvre en plaquettes (PPP)
    100 µl de tampon Owren Koller (TOK)

100 µl de réactif APTT
incubation 3 min à 37°C
100 µl de chlorure de calcium (CaCl$_2$) 25 mM
mesure du temps d'apparition du caillot.

Une droite est tracée en portant le logarithme du temps de coagulation en fonction de la concentration du polymère. La pente de la droite permet de calculer l'activité anticoagulante spécifique du polysaccharide d'après l'équation :

$$a(\text{UI/mg}) = \frac{\text{pente de la droite DCMBSu}}{\text{pente de la droite héparine}} \times \text{activité anticoagulante spécifique de l'héparine étalon utilisée}$$

### b) Mesure de l'inhibition de l'activation du complément

L'action de dérivés de dextrane de compositions chimiques variables a été étudiée dans un dosage hémolytique ou CH50, selon un protocole dans lequel le sérum humain est incubé avec l'un des dérivés du dextrane de l'invention pendant 30 minutes à 37°C avant d'être activé par des érythrocytes de mouton sensibilisés par des anticorps de lapin antiglobules rouges de mouton (EA). Par définition, une unité CH50 correspond à la concentration de protéines du complément (contenue dans un millilitre de sérum) capable d'induire l'hémolyse de 50% de $2 \times 10^7$ EA activés dans un milieu réactionnel où sont maintenus constants le volume, la température et le temps de réaction. Le nombre de sites hémolytiques par cellule est calculé.

L'activité inhibitrice de l'activation du complément est exprimée en pourcentage d'inhibition de formation de convertase par référence au tube témoin où le nombre de sites hémolytiques est déterminé en l'absence de dérivé de dextrane.

Cette activité, A, est exprimée en poids de produit (à volume constant) (µg), nécessaire à 50% d'inhibition de formation de sites hémolytiques. Cette expression de l'activité anticomplémentaire signifie que celle-ci est inversement proportionnelle au poids de produit. En d'autres termes, l'activité anticomplémentaire est d'autant plus élevée que le poids de produit est plus faible.

Cette activité varie en fonction de la composition chimique globale du produit.

Les résultats obtenus avec 5 dérivés selon l'invention sont illustrés dans le Tableau II ci-après, qui rappelle leur composition chimique globale et leur activité anticoagulante spécifique, a, exprimée en unité internationale par mg de produit (par référence à l'héparine à 173 UI/mg).

TABLEAU II

| Référence de l'échantillon | Composition chimique | | | Activité anti-complémentaire | Activité anti- coagulante |
|---|---|---|---|---|---|
| | CM | B | Su | A µg | a UI/mg |
| Dextrane | 0 | 0 | 0 | 1250 | 0 |
| Dextrane CM | 1,0 | 0 | 0 | 570 | 0 |
| DCMBSu1 | 0,75 | 0,20 | 0,15 | 150 | 0,02 |
| DCMBSu2 | 0,80 | 0,25 | 0,15 | 150 | 0,04 |
| DCMBSu3 | 1,10 | 0,40 | 0,40 | 35 | 4,0 |
| DCMBSu4 | 0,70 | 0,30 | 0,25 | 125 | 0,08 |
| DCMB1 | 0,80 | 0,10 | 0 | 300 | 0 |
| Héparine | - | - | - | 2700 | 173 |

On constate que l'activité anti-complémentaire croît en même temps que l'augmentation du ds en motifs CM et Su. Au delà d'un ds en motifs CM de l'ordre de 0,40, l'activité anticoagulante croît en même temps que l'augmentation du ds en motifs Su.

On observe que les dérivés de faible ds en motifs Su présentent déjà une activité anticomplémentaire importante alors que leur activité anticoagulante est très faible. On remarque enfin que la seule présence de motifs CM et B induit une légère activité anti-complémentaire alors qu'elle n'entraîne aucun effet anti-coagulant.

Ces résultats montrent qu'il est possible d'obtenir des produits dotés d'une forte activité d'inhibition de l'activation du complément et d'une activité anti-coagulante spécifique très faible.

**EXEMPLE 7 : Activité antiproliférative des composés selon l'invention.**

Les composés DCMBSu ont des effets de stimulation ou d'inhibition de la croissance cellulaire, selon la nature des cellules. Ainsi, par exemple dans le domaine cardio-vasculaire, les produits présentent l'avantage de stimuler la croissance des cellules endothéliales et d'inhiber la croissance des cellules musculaires lisses (CML). Ces propriétés sont importantes en pharmacologie, leur intérêt majeur étant la prévention de la resténose après angioplastie ou interventions chirurgicales cardio-vasculaires.

L'activité antiproliférative des DCMBS**u** a été étudiée sur les cellules musculaires lisses de l'aorte de rat (CML).

24 heures après l'ensemencement des cellules dans un milieu à 10% de sérum de veau foetal (SVF), les cellules sont mises 3 jours en carence (milieu à 0,1% de SVF) (cela permet aux cellules de se synchroniser en les stoppant au stade GO/G1 du cycle cellulaire). Les cellules sont ensuite réalimentées par du milieu à 10% de SVF contenant également des concentrations variables du produit à tester (de 0 à 1 mg/ml). Un témoin est réalisé dans les mêmes conditions mais sans ajout de produit.

Au bout de 5 jours de croissance, les cellules sont comptées (à l'aide d'un compteur automatique ou par mesure de la radioactivité en coups par minute après incorporation de thymidine tritiée) et l'inhibition est déterminée comme suit, les initiales « nb » désignant le mot « nombre » :

$$I\% = (1 - (\text{nb de cellules en présence de produit})/(\text{nb de cellules sans produit})) \times 100$$

L'activité anticoagulante des différents produits a également été déterminée.

Le Tableau III ci-dessous résume les résultats obtenus pour deux DCMBSu ainsi que pour le dextrane natif et l'héparine.

TABLEAU III

| Produit | Composition (ds) | | | Activité anti-coagulante | 1 |
|---|---|---|---|---|---|
| | CM | B | Su | (UI/mg) | (%) |
| Dextrane | 0 | 0 | 0 | 0 | 0 |
| DCMBSu6 | 0,60 | 0,50 | 0,30 | 5,0 | 80 |
| DCMBSu5 | 0,80 | 0,35 | 0,15 | 3,0 | 85 |
| Héparine | | | | 173 | 80 |

Ces résultats montrent que pour les DCMBSu6 et DCMBSu5, l'inhibition (I) de la croissance cellulaire des cellules musculaires lisses est comparable, voire supérieure, à celle de l'héparine. Cependant, ces deux produits présentent une activité anticoagulante nettement inférieure à celle de l'héparine.

**EXEMPLE 8 : Potentialisation de la croissance cellulaire en présence de facteurs de croissances *Fibroblast Growth Factor* (FGF).**

L'évaluation de l'activité proliférative des DCMBSu a été réalisée sur 2 lignées cellulaires :

Les CCL39 : lignée fibroblastique de poumon de hamster chinois.

Les CEVCOH : cellules endothéliales de la veine du cordon ombilicale humain.

Les essais de croissance sont effectués en maintenant les cellules en carence, c'est-à-dire dans un milieu de culture contenant 0% ou 2 % de sérum de veau foetal, puis en incorporant le produit à étudier à différentes concentrations.

Les comptages sont effectués à l'aide d'un compteur automatique ou par mesure de la radioactivité en coups par minute après incorporation de thymidine tritiée.

La capacité des produits à potentialiser et à protéger certains facteurs de croissance comme les FGF est évaluée selon le protocole suivant.

L'activité mitogénique des FGF étudiés est mesurée par un test d'activité biologique (test dose réponse) afin de déterminer la DE 50 (quantité de facteur de croissance nécessaire pour obtenir la moitié de l'incorporation maximale de la thymidine tritiée par les cellules).

Cette DE 50 est de 5 ng/ml pour les CCL39 et de 2 ng/ml pour les CEVCOH.

Ces valeurs de DE 50 sont utilisées pour la suite des études.

Pour les CEVCOH, l'association FGF (2 ng/ml) avec 400 pg/ml de DCMBSu5 a le même pouvoir mitogénique que le FGF seul à 20 ng/ml.

Pour les CCL39, en absence de DCMBSu la DE 50 est de l'ordre de 5 ng/ml de FGF. En présence de 1000 pg/ml de DCMBSul, la DE 50 diminue à 0,8 ng/ml.

Ces résultats montrent l'effet de potentialisation des facteurs de croissance cellulaire considérés par les DCMBSu. Cet effet est lié à la composition chimique globale des produits. Le Tableau suivant donne, par exemple, la composition chimique globale de DCMBSu particulièrement actifs.

| COMPOSITIONS DES DCMBSu (en ds) | | | |
|---|---|---|---|
| | CM | B | Su |
| **DCMBSu1** | 0,75 | 0,20 | 0,15 |
| **DCMBSu5** | 0,80 | 0,35 | 0,15 |
| **DCMBSu3** | 1,10 | 0,40 | 0,40 |

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée, de la présente invention.

**Revendications**

1. Dérivés de dextrane de formule générale $DCM_aB_bSu_cS_d$, dans laquelle :

   D représente une chaîne polysaccharidique, de préférence constituée par des enchaînements d'unités glucosidiques,
   CM représente des groupes carboxyméthyle,
   B représente des groupes carboxyméthylbenzylamides,
   Su représente des groupes sulfates,
   S représente des groupes sulfonates,
   a, b, c et d représentent le degré de substitution (ds), exprimé par rapport au nombre de fonctions hydroxyles libres dans une unité glucosidique du dextrane, respectivement en groupements CM, B, Su et S ; a étant $\geq$ à 0,3, b étant égal à 0 ou $\geq$ à 0,1, c étant égal à 0 ou $\geq$ à 0,1 et d étant égal à 0 ou $\leq$ à 0,15, à condition que lorsque d=0, a et b sont $\neq$ 0,

   lesquels produits présentent :

   - une homogénéité de la distribution des tailles de chaînes, illustrée par un profil d'élution de type gaussien symétrique en chromatographie d'exclusion stérique haute performance, et
   - une homogénéité de la distribution des groupements chimiques chargés, illustrée par un profil d'élution à un seul pic symétrique en chromatographie d'échange d'ions basse pression.

2. Dérivés de dextrane, de formule générale $DCM_aB_bSu_cS_d$, selon la revendication 1, **caractérisé en ce que** a $\geq$ 0,6, b $\neq$ 0, c égal à 0 ou $\leq$ 0,5 et d $\leq$ 0,15 ou égal à 0 et **en ce que** leur masse molaire est comprise entre 3.000 et 500.000 g/mole, pour une utilisation essentiellement comme agent cicatrisant.

3. Dérivés de dextrane, de formule générale $DCM_aB_bSu_cS_d$, selon la revendication 1, **caractérisés en ce que** a $\geq$ 0,3, b $\neq$ 0, c égal à 0 ou $\leq$ 0,4 et d $\leq$ 0,15 ou égal à 0 et **en ce que** leur masse molaire est comprise entre 10.000 et 60.000 g/mole, pour une utilisation essentiellement comme agent à activité anti-complémentaire et substitut du plasma.

4. Dérivés de dextrane, de formule générale $DCM_aB_bSu_cS_d$, selon la revendication 1, **caractérisés en ce que** a $\geq$ 0,5, b $\neq$ 0, c égal à 0 ou $\leq$ 0,4 et d $\leq$ 0,15 ou égal à 0 et **en ce que** leur masse molaire est comprise entre 3.000 et 100.000 g/mole, pour une utilisation essentiellement comme agent modulateur de la prolifération cellulaire.

**5.** Dérivés de dextrane, de formule générale DCM$_a$B$_b$Su$_c$S$_d$, selon la revendication 1, **caractérisés en ce que** $\underline{a} \geq$ 0,4, $\underline{b} \neq 0$, $\underline{c} \geq 0,3$ et $\underline{d} \leq 0,15$ ou égal à 0 et **en ce que** leur masse molaire est comprise entre 3.000 et 20.000 g/ mole, pour une utilisation essentiellement comme agent anticoagulant.

**6.** Médicaments, **caractérisés en ce qu'**ils comprennent comme principe actif au moins un dérivé de dextrane selon l'une quelconque des revendications 1 à 5, éventuellement associé à un autre principe actif et/ou à au moins un véhicule pharmaceutiquement acceptable et/ou un support physiologiquement acceptable, de préférence un liposome.

**7.** Procédé de préparation de dérivés du dextrane de formule générale DCM$_a$B$_b$Su$_c$,S$_d$ selon l'une quelconque des revendication 1 à 5, **caractérisé en ce qu'**il comprend les étapes suivantes :

a) **carboxyméthylation** comprenant (i) l'activation d'un dextrane non substitué, par mise en contact dudit dextrane avec un milieu hydro-alcoolique liquide biphasique basique pendant au moins 1 h sous agitation, (ii) addition de l'acide monochloroacétique au produit activé obtenu, à une température comprise entre 40 et 90°C, de préférence à 60°C, le rapport R$_{CM}$, égal au nombre de moles d'acide monochloracétique/nombre de moles d'OH étant compris entre 0,3 et 2, (iii) isolement et éventuellement purification du dextrane carboxyméthyle (DCM) obtenu.

b) **couplage de la benzylamine sur les groupes carboxyméthyle** comprenant (i) la mise en contact, pendant au moins 2 h et en milieu aqueux acide, du DCM obtenu en a) avec de la benzylamine, en présence d'une carbodiimide hydrosoluble comme agent de couplage, à une température comprise entre 0°C et 30°C, le rapport molaire CMC/CM étant compris entre 0,25 et 2 et le rapport molaire benzylamine/CM étant compris entre 0,25 et 2, (ii) l'isolement du dextrane méthylcarboxyle benzylamide (DCMB) obtenu et éventuellement sa purification.

c) **sulfatation** comprenant (i) la formation d'un sel de trialkylammonium du DCMB obtenu en b), (ii) la solubilisation du sel obtenu dans un solvant polaire anhydre, généralement une base de Lewis, telle que le diméthylsulfoxyde (DMSO) ou la diméthylformamide (DMF) et (iii) l'ajout audit sel en solution, d'un complexe à base de trioxyde de soufre tel que SO$_3$-pyridine, SO$_3$-triéthylamine ou SO$_3$-DMF en solution dans le même solvant, à une température inférieure à 70°C, le rapport molaire complexe à base de trioxyde de soufre/OH libres étant compris entre 0,25 et 12, et éventuellement

d) **sulfonatation** des groupes B par mélange sous agitation d'un dérivé DCMBSu en suspension dans un solvant anhydre avec de l'acide chlorosulfonique en solution dans le même solvant, à une température comprise entre la température ambiante et la température d'ébullition du solvant utilisé.

**8.** Procédé selon la revendication 7, **caractérisé en ce qu'**à l'étape a), le rapport eau:alcool dudit milieu hydroalcoolique liquide biphasique est compris entre 10:90 (v/v) et 25:75 (v/v), et est de préférence de 15:85 (v/v).

**9.** Procédé selon la revendication 7 ou la revendication 8, **caractérisé en ce que** la carbodiimide hydrosoluble de l'étape b) est sélectionnée dans le groupe constitué par le 1-cyclohexyl-3-(2-morpholinoéthyl)-carbodiimide méta-*p*-toluène sulfonate (CMC) et le chlorhydrate de 1-éthyl-3-(3-diméthylamino-propyl)-carbodiimide (EDC).

**10.** Procédé de préparation des dérivés de dextrane selon l'une quelconque des revendications 1 à 5, de formule générale DCM$_a$B$_b$Su$_c$S$_d$, dans laquelle a $\neq$ 0, $\underline{b}$ est différent de 0 et $\underline{d}$ = 0, **caractérisé en ce qu'**il comprend les étapes suivantes :

a) préparation de N-méthyl-phényl-2-chloroacétamide par mise en contact d'une benzylamine avec du chlorure de chloroacétyle, puis isolement et purification du produit,
b) mise en contact du dextrane en solution hydro-alcoolique basique avec successivement le N-méthyl-phényl-2-chloroacétamide en solution alcoolique obtenu en a) en présence d'acide monochloroacétique en solution alcoolique, maintien du mélange obtenu à une température supérieure à 40°C, de préférence à 60°C, puis isolement et éventuellement purification du DCMB obtenu, et
c) sulfatation du produit obtenu en b) comprenant (i) la formation d'un sel de trialkylammonium, (ii) la solubilisation du sel obtenu dans un solvant polaire anhydre, généralement une base de LEWIS, telle que le diméthylsulfoxyde (DMSO) ou la diméthylformamide (DMF), (iii) l'ajout audit sel en solution d'un complexe à base de trioxyde de soufre tel que SO$_3$-pyridine, SO$_3$-triéthylamine ou SO$_3$-DMF en solution dans le même solvant, à température inférieure à 70°C, le rapport molaire complexe à base de trioxyde de soufre/OH libres étant compris entre 0,25 et 12 et (iv) l'isolement et éventuellement la purification du DCMBSu obtenu.

**11.** Procédé de préparation des dérivés de dextrane de formule générale $DCM_aB_bSu_cS_d$, selon la revendication 1, dans laquelle $\underline{a}$ est différent de 0, $\underline{b}$ = 0 et $\underline{d}$ = 0, **caractérisé en ce qu'**il comprend les étapes suivantes :

 a) **carboxyméthylation** d'un dextrane non substitué, dans les conditions exposées à la revendication 7, et
 b) **sulfatation** du DCM obtenu en a) dans les conditions exposées à la revendication 7.

**12.** Dextrane carboxyméthyl benzylamide de formule $DCM_aB_b$ dans le cas où $\underline{a} \neq 0$ et $\underline{b} \neq 0$, en tant que produit intermédiaire pour la préparation d'un dérivé de dextrane de formule générale $DCM_aB_bSu_cS_d$ selon la revendication 1.

**13.** Dextrane benzylamide (DB) en tant que produit intermédiaire pour la préparation d'un dérivé de dextrane de formule générale $DCM_aB_bSu_cS_d$ selon la revendication 1.

**14.** Dérivé du dextrane selon la revendication 12, pour une utilisation comme agent à activité anti-complémentaire.

**Claims**

**1.** Dextran derivatives of general formula $DCM_aB_bSu_cS_d$, in which:

 D represents a polysaccharide chain, preferably consisting of concatenations of glucoside units,
 CM represents carboxymethyl groups,
 B represents carboxymethylbenzylamide groups,
 Su represents sulphate groups,
 S represents sulphonate groups,
 $\underline{a}$, $\underline{b}$, $\underline{c}$ and $\underline{d}$ represent the degree of substitution (ds), expressed relative to the number of free hydroxyl functions in a dextran glucoside unit, with groups CM, B, Su and S, respectively; $\underline{a}$ being $\geq 0.3$, $\underline{b}$ being equal to 0 or $\geq 0.1$, $\underline{c}$ being equal to 0 or $\geq 0.1$ and $\underline{d}$ being equal to 0 or $\leq 0.15$, with the proviso that when $\underline{d}$ = 0, $\underline{a}$ and $\underline{b}$ are $\neq 0$,

 which products display:

 - homogeneity of the size distribution of the chains, illustrated by an elution profile of symmetrical Gaussian type in high performance steric exclusion chromatography, and
 - homogeneity of the distribution of charged chemical groups, illustrated by an elution profile as a single symmetrical peak in low-pressure ion exchange chromatography.

**2.** Dextran derivatives of general formula $DCM_aB_bSu_cS_d$ according to Claim 1, **characterized in that** $\underline{a} \geq 0.6$, $\underline{b} \neq 0$, $\underline{c}$ equal to 0 or $\leq 0.5$ and $\underline{d} \leq 0.15$ or equal to 0 and **in that** their molar mass is between 3000 and 500,000 g/mol, for use essentially as cicatrizing agents.

**3.** Dextran derivatives of general formula $DCM_aB_bSu_cS_d$ according to Claim 1, **characterized in that** $\underline{a} \geq 0.3$, $\underline{b} \neq 0$, $\underline{c}$ equal to 0 or $\leq 0.4$ and $\underline{d} \leq 0.15$ or equal to 0 and **in that** their molar mass is between 10,000 and 60,000 g/mol, for use essentially as agents with anti-complement activity and as substitutes for plasma.

**4.** Dextran derivatives of general formula $DCM_aB_bSu_cS_d$ according to Claim 1, **characterized in that** $\underline{a} \geq 0.5$, $\underline{b} \neq 0$, $\underline{c}$ equal to 0 or $\leq 0.4$ and $\underline{d} \leq 0.15$ or equal to 0 and **in that** their molar mass is between 3000 and 100,000 g/mol, for use essentially as agents for modifying cell proliferation.

**5.** Dextran derivatives, of general formula $DCM_aB_bSu_cS_d$ according to Claim 1, **characterized in that** $\underline{a} \geq 0.4$, $\underline{b} \neq 0$, $\underline{c} \geq 0.3$ and $\underline{d} \leq 0.15$ or equal to 0 and **in that** their molar mass is between 3000 and 20,000 g/mol, for use essentially as anticoagulant agents.

**6.** Medicines, **characterized in that** they comprise, as active principle, at least one dextran derivative according to any one of Claims 1 to 5, optionally combined with another active principle and/or with at least one pharmaceutically acceptable vehicle and/or a physiologically acceptable support, preferably a liposome.

**7.** Process for preparing dextran derivatives of general formula $DCM_aB_bSu_cS_d$ according to any one of Claims 1 to 5, **characterized in that** it comprises the following steps:

a) **carboxymethylation** comprising (i) activation of an unsubstituted dextran, by placing said dextran in contact with a basic two-phase liquid aqueous-alcoholic medium for at least 1 h with stirring, (ii) addition of monochloroacetic acid to the activated product obtained, at a temperature of between 40 and 90°C, preferably at 60°C, the ratio $R_{CM}$, equal to the number of moles of monochloroacetic acid/number of moles of OH, being between 0.3 and 2, (iii) isolation and optionally purification of the dextran carboxymethyl (DCM) obtained.

b) **coupling of benzylamine with carboxymethyl groups** comprising (i) the placing in contact, for at least 2 h and in an acidic aqueous medium, of the DCM obtained in a) with benzylamine, in the presence of a water-soluble carbodiimide as coupling agent, at a temperature of between 0°C and 30°C, the CMC/CM molar ratio being between 0.25 and 2 and the benzylamine/CM molar ratio being between 0.25 and 2, (ii) isolation of the dextran methylcarboxyl benzylamide (DCMB) obtained and optionally purification thereof.

c) **sulphation** comprising (i) the formation of a trialkylammonium salt of the DCMB obtained in b), (ii) solubilization of the salt obtained in an anhydrous polar solvent, generally a Lewis base, such as dimethyl sulphoxide (DMSO) or dimethylformamide (DMF) and (iii) addition, to said dissolved salt, of a complex based on sulphur trioxide such as $SO_3$-pyridine, $SO_3$-triethylamine or $SO_3$-DMF dissolved in the same solvent, at a temperature of less than 70°C, the complex based on sulphur trioxide/free OH molar ratio being between 0.25 and 12, and optionally

d) **sulphonation** of the groups B by mixing, with stirring, a derivative DCMBSu in suspension in an anhydrous solvent with chlorosulphonic acid dissolved in the same solvent, at a temperature between room temperature and the boiling point of the solvent used.

8. Process according to Claim 7, **characterized in that**, in step a), the water/alcohol ratio in said two-phase liquid aqueous-alcoholic medium is between 10/90 (v/v) and 25/75 (v/v) and is preferably 15/85 (v/v).

9. Process according to Claim 7 or Claim 8, **characterized in that** the water-soluble carbodiimide from step b) is selected from the group consisting of 1-cyclohexyl-3-(2-morpholinoethyl)carbodiimide meta-p-toluenesulphonate (CMC) and 1-ethyl-3-(3-dimethylaminopropyl)carbodiimide hydrochloride (EDC).

10. Process for preparing the dextran derivatives according to any one of Claims 1 to 5, of general formula $DCM_aB_b$-$Su_cS_d$, in which $\underline{a} \neq 0$, $\underline{b}$ is other than 0 and $\underline{d} = 0$, **characterized in that** it comprises the following steps:

a) preparation of N-methylphenyl-2-chloroacetamide by placing a benzylamine in contact with chloroacetyl chloride, followed by isolation and purification of the product,

b) placing dextran, dissolved in basic aqueous-alcoholic solution, in contact successively with N-methylphenyl-2-chloroacetamide in alcoholic solution obtained in a) in the presence of monochloroacetic acid in alcoholic solution, maintaining the mixture obtained at a temperature above 40°C, preferably at 60°C, followed by isolation and optionally purification of the DCMB obtained, and

c) sulphation of the product obtained in b) comprising (i) formation of a trialkylammonium salt, (ii) dissolution of the salt obtained in an anhydrous polar solvent, generally a Lewis base, such as dimethyl sulphoxide (DMSO) or dimethylformamide (DMF), (iii) addition, to said dissolved salt, of a complex based on sulphur trioxide such as $SO_3$-pyridine, $SO_3$-triethylamine or $SO_3$-DMF dissolved in the same solvent, at a temperature below 70°C, the complex based on sulphur trioxide/free OH molar ratio being between 0.25 and 12, and (iv) isolation and optionally purification of the DCMBSu obtained.

11. Process for preparing the dextran derivatives of general formula $OCM_aB_bSu_cS_d$ according to Claim 1, in which $\underline{a}$ is other than 0, $\underline{b} = 0$ and $\underline{d} = 0$, **characterized in that** it comprises the following steps:

a) **carboxymethylation** of an unsubstituted dextran, under the conditions outlined in Claim 7, and

b) **sulphation** of the DCM obtained in a) under the conditions outlined in Claim 7.

b) **sulphation** of the DCM obtained in a) under the conditions outlined in Claim 7.

12. Dextran carboxymethyl benzylamide of formula $DCM_aB_b$, in the case in which $\underline{a} \neq 0$ and $\underline{b} \neq 0$, as an intermediate product for the preparation of a dextran derivative of general formula $DCM_aB_bSu_cS_d$ according to Claim 1.

13. Dextran benzylamide (DB), as an intermediate product for the preparation of a dextran derivative of general formula $DCM_aB_bSu_cS_d$ according to Claim 1.

**14.** Dextran derivative according to Claim 12, for use as an agent with anti-complement activity.

**Patentansprüche**

**1.** Dextranderivate mit der allgemeinen Formel $DCM_aB_bSu_cS_d$, wobei

D eine Polysaccharidkette darstellt, die vorzugsweise durch Verkettungen von Glucoseeinheiten gebildet ist,
CM Carboxymethylgruppen darstellt,
B Carboxymethylbenzylamidgruppen darstellt,
Su Sulfatgruppen darstellt,
S Sulfonatgruppen darstellt,
$\underline{a}$, $\underline{b}$, $\underline{c}$ und $\underline{d}$ den Substitutionsgrad (ds) darstellen, ausgedrückt im Verhältnis zur Anzahl freier Hydroxylfunktionen in einer Glucoseeinheit des Dextrans in den Gruppierungen CM, B, Su bzw. S; wobei $\underline{a} \geq 0{,}3$, $\underline{b} = 0$ oder $\geq 0{,}1$, $\underline{c} = 0$ oder $\geq 0{,}1$ und $\underline{d} = 0$ oder $\leq 0{,}15$, unter der Bedingung, dass $\underline{d} = 0$, $\underline{a}$ und $\underline{b} \neq 0$,
welche Produkte aufweisen:

- eine Gleichmäßigkeit der Verteilung der Kettengrößen, die sich durch ein symmetrisches Gauß'sches Eluierungsprofil in leistungsstarker sterischer Ausschlusschromatographie darstellt, und
- eine Gleichmäßigkeit der Verteilung der geladenen chemischen Gruppen, die sich durch ein symmetrisches Eluierungsprofil mit einer einzigen Spitze in Niederdruckionenaustauschchromatographie darstellt.

**2.** Dextranderivate mit der allgemeinen Formel $DCM_aB_bSu_cS_d$, nach Anspruch 1, **dadurch gekennzeichnet, dass** $\underline{a} \geq 0{,}6$, $\underline{b} \neq 0$, $\underline{c} = 0$ oder $\leq 0{,}5$ und $\underline{d} \leq 0{,}15$ oder $= 0$, und dass ihre molare Masse 3.000 bis 500.000 g/Mol beträgt, für eine Verwendung im Wesentlichen als Wundheilungsmittel.

**3.** Dextranderivate mit der allgemeinen Formel $DCM_aB_bSu_cS_d$, nach Anspruch 1, **dadurch gekennzeichnet, dass** $\underline{a} \geq 0{,}3$, $\underline{b} \neq 0$, $\underline{c} = 0$ oder $\leq 0{,}4$ und $\underline{d} \leq 0{,}15$ oder $= 0$, und dass ihre molare Masse 10.000 bis 60.000 g/Mol beträgt, für eine Verwendung im Wesentlichen als Mittel mit antikomplementärer und Plasma ersetzender Wirkung.

**4.** Dextranderivate mit der allgemeinen Formel $DCM_aB_bSu_cS_d$, nach Anspruch 1, **dadurch gekennzeichnet, dass** $\underline{a} \geq 0{,}5$, $\underline{b} \neq 0$, $\underline{c} = 0$ oder $\leq 0{,}4$ und $\underline{d} \leq 0{,}15$ oder $= 0$, und dass ihre molare Masse 3.000 bis 100.000 g/Mol beträgt, für eine Verwendung im Wesentlichen als Modulatormittel für die Zellvermehrung.

**5.** Dextranderivate mit der allgemeinen Formel. $DCM_aB_bSu_cS_d$, nach Anspruch 1, **dadurch gekennzeichnet, dass** $\underline{a} \geq 0{,}4$, $\underline{b} \neq 0$, $\underline{c} \geq 0{,}3$ und $\underline{d} \leq 0{,}15$ oder $= 0$, und dass ihre molare Masse 3.000 bis 20.000 g/Mol beträgt, für eine Verwendung im Wesentlichen als Antikoagulans.

**6.** Medikamente, **dadurch gekennzeichnet, dass** sie als Wirkstoff mindestens ein Dextranderivat nach einem der Ansprüche 1 bis 5 umfassen, das gegebenenfalls mit einem anderen Wirkstoff und/oder mit mindestens einem pharmazeutisch zugelassenen Arzneistoffträger und/oder einem physiologisch annehmbaren Träger, vorzugsweise einem Liposom, verbunden ist.

**7.** Herstellungsverfahren für Dextranderivate mit der allgemeinen Formel $DCM_aB_bSu_cS_d$, nach einem der Ansprüche 1 bis 5 , **dadurch gekennzeichnet, dass** es die folgenden Schritte umfasst:

a) **Carboxymethylierung**, umfassend (i) die Aktivierung eines nicht substituierten Dextrans, indem dieses Dextran unter Rühren mindestens 1 Stunde lang mit einem flüssigen, zweiphasigen, basischen Wasser-/Alkoholmedium in Kontakt gebracht wird, (ii) Zugabe von Monochloressigsäure zum erhaltenen aktivierten Produkt, bei einer Temperatur von 40 bis 90°C, vorzugsweise 60°C, wobei das Verhältnis $R_{CM}$, das gleich der Molanzahl der Monochloressigsäure/Molanzahl von OH ist, 0,3 bis 2 beträgt, (iii) Isolierung und gegebenenfalls Reinigung des erhaltenen Carboxymethyldextrans (DCM).
b) **Binden des Benzylamins an die Carboxymethylgruppen,** umfassend, (i) das in a) erhaltene DCM mindestens 2 Stunden lang und in saurem wässrigen Medium im Beisein eines wasserlöslichen Carbodiimids als Bindemittel bei einer Temperatur von 0°C bis 30°C mit Benzylamin in Kontakt zu bringen, wobei das Molverhältnis CMC/CM 0,25 bis 2 und das Molverhältnis Benzylamin/CM 0,25 bis 2 beträgt; (ii) Isolierung und gegebenenfalls Reinigung des erhaltenen Benzylamidmethylcarboxyldextrans (DCMB),
c) **Sulfatierung,** umfassend, (i) Bildung eines Trialkylammoniumsalzes des in b) erhaltenen DCMBs, (ii) Löslichmachen des erhaltenen Salzes in einem wasserfreien polaren Lösungsmittel, im Allgemeinen einer Lewis-Base wie Dimethylsulfoxid (DMSO) oder Dimethylformamid (DMF), und (iii) Zugabe zu dem in Lösung befindlichen Salz eines Komplexes auf der Basis von Schwefeltrioxid, wie $SO_3$-Pyridin, $SO_3$-Triethylamin oder $SO_3$-DMF in Lösung im selben Lösungsmittel, bei einer Temperatur unter 70°C, wobei das Molverhältnis des Komplexes auf der Basis von Schwefeltrioxid/freies OH 0,25 bis 12 beträgt, und gegebenenfalls

d) **Sulfonierung** der B-Gruppen durch Mischen unter Rühren eines DCMBSu-Derivats, das sich in Suspension in einem wasserfreien Lösungsmittel befindet, mit Chlorsulfonsäure, die im selben Lösungsmittel gelöst ist, bei einer Temperatur zwischen Raumtemperatur und der Siedetemperatur des verwendeten Lösungsmittels.

8. Verfahren nach Anspruch 7, **dadurch gekennzeichnet, dass** beim Schritt a) das Verhältnis Wasser : Alkohol des flüssigen zweiphasigen Wasser-/Alkoholmediums 10 : 90 (v/v) bis 25 : 75 (v/v), und vorzugsweise 15 : 85 (v/v) beträgt.

9. Verfahren nach Anspruch 7 oder 8, **dadurch gekennzeichnet, dass** das wasserlösliche Carbodiimid des Schritts b) aus der Gruppe ausgewählt ist, die aus 1-Cyclohexyl-3-(2-morpholinoethyl)-carbodiimidmeta-p-toluolsulfonat (CMC) und dem Chlorhydrat von 1-Ethyl-3-(3-dimethylaminopropyl)-carbodiimid (EDC) besteht.

10. Herstellungsverfahren für Dextranderivate nach einem der Ansprüche 1 bis 5 mit der allgemeinen Formel $DCM_aB_b$-$Su_cS_d$, wobei $\underline{a} \neq 0$, $\underline{b} \neq 0$ und $\underline{d} = 0$, **dadurch gekennzeichnet, dass** es die folgenden Schritte umfasst:

   a) Zubereiten von N-methylphenyl-2-chloracetamin, indem Benzylamin mit Chloracetylchlorid in Kontakt gebracht wird, dann Isolierung und Reinigung des Produkts,
   b) das in basischer Wasser-/Alkohollösung befindliche Dextran nacheinander mit dem bei a) erhaltenen, in Alkohollösung befindlichen N-methylphenyl-2-chloracetamin im Beisein von in Alkohollösung befindlicher Monochloressigsäure in Kontakt zu bringen, das erhaltene Gemisch auf einer Temperatur über 40°C, vorzugsweise auf 60°C zu halten, dann Isolierung und gegebenenfalls Reinigung des erhaltenen DCMBs, und
   c) Sulfatierung des bei b) erhaltenen Produkts, umfassend (i) Bildung eines Trialkylammoniumsalzes, (ii) Löslichmachen des erhaltenen Salzes in einem wasserfreien polaren Lösungsmittel, im Allgemeinen einer Lewis-Base wie Dimethylsulfoxid (DMSO) oder Dimethylformamid (DMF), und (iii) Zugabe zu dem in Lösung befindlichen Salz eines Komplexes auf der Basis von Schwefeltrioxid, wie $SO_3$-Pyridin, $SO_3$-Triethylamin oder $SO_3$-DMF in Lösung im selben Lösungsmittel, bei einer Temperatur unter 70°C, wobei das Molverhältnis des Komplexes auf der Basis von Schwefeltrioxid/freies OH 0,25 bis 12 beträgt, und (iv) Isolierung und gegebenenfalls Reinigung des erhaltenen DCMBSu

11. Herstellungsverfahren für Dextranderivate nach Anspruch 1 mit der allgemeinen Formel $DCM_aB_bSu_cS_d$, wobei $\underline{a} \neq 0$, $\underline{b} = 0$ und $\underline{d} = 0$, **dadurch gekennzeichnet, dass** es die folgenden Schritte umfasst:

   a) **Carboxymethylierung** eines nicht substituierten Dextrans unter den in Anspruch 7 dargelegten Bedingungen, und
   b) **Sulfatierung** des in a) erhaltenen DCM unter den in Anspruch 7 dargelegten Bedingungen.

12. Carboxymethylbenzylamiddextran mit der Formel $DCM_aB_b$, bei dem $\underline{a} \neq 0$ und $\underline{b} \neq 0$, als Zwischenprodukt für die Herstellung eines Dextranderivats mit der allgemeinen Formel $DCM_aB_bSu_cS_d$ nach Anspruch 1.

13. Benzylamiddextran (DB) als Zwischenprodukt für die Herstellung eines Dextranderivats mit der allgemeinen Formel $DCM_aB_bSu_cS_d$ nach Anspruch 1.

14. Dextranderivat nach Anspruch 12, für eine Verwendung als Mittel mit antikomplementärer Wirkung.

D          CM          B          Su          S

EP 0 990 002 B1

**FIGURE 1**

FIGURE 2

FIGURE 3

EP 0 990 002 B1